# EUROPEAN PATENT APPLICATION

(11) **EP 2 687 852 A1**
(43) Date of publication of application: **22.01.2014**
(21) Application number: 12382283.5
(22) Date of filing: 17.07.2012
(51) Int. Cl.: G01N 33/68

(54) **Method for diagnosing and treating chronic fatigue syndrome**

(71) Applicant: Laboratorios del. Dr. Esteve, S.A., 08041 Barcelona (ES); Fundació Privada Institut de Recerca de la SIDA-Caixa, 08916 Badalona (ES)
(72) Inventor: Cabrera Navarro, Cecilia, 08440 Cardedeu, Barcelona (ES); Curriu Marti, Marta, 43004 Tarragona (ES); Massanella Luna, Marta, 08960 Sant Just Desvern (ES); Carrillo Molina, Jorge, 08915 Badalona (ES); Blanco Arbues, Julian Miguel, 08500 Vic (ES)
(74) Representative: ABG Patentes, S.L.

(57) **Abstract**

The present invention relates to a method for diagnosing CFS or a predisposition thereto *in vitro* which comprises determining the levels of CD25, CD69, NKp46 and CD57 biomarkers in CD56+CD16+ NK cells and comparing with a reference value wherein increased levels of said markers is indicative the subject suffers or is predisposed to suffering CFS. Likewise the invention relates to a kit comprising reagents for assaying the levels of CD25, CD69, NKp46 and CD57 in CD56+CD16+ NK cells and to the use of said kit for the diagnosis of CFS or of a predisposition thereto. In addition, the invention relates to a reagent for inhibiting the expression or activity of at least one biomarker selected from the group consisting of CD69, NKp46 and CD5 or to a reagent for inducing the expression or activity of at least one biomarker selected from CD25, CD57, CD56 and Ki67 for use in the treatment or prevention of CFS in a subject in need thereof. Finally the invention relates to a kit comprising a reagent for inhibiting or inducing a biomarker of the invention and to the use of said kit for the treatment or prevention of CFS.

## Description

### Field of the Invention

The present invention refers to methods and kits for diagnosing and treating chronic fatigue syndrome.

### Background of the Invention

The chronic fatigue syndrome (CFS) is a complex clinical condition of unknown etiology, characterized by persistent or intermittent fatigue that is not the result of recent exertion and does not improve with rest, resulting in a significant reduction in the patient's previous normal activity. *See* Fukuda K, et al., Ann. Intern. Med. 1994; 121: 953-959. The disease is widely prevalent among the general population. There are more than 1 million CFS patients in the United States alone. HIV patients are afflicted particularly with CFS (HIV-related CFS). At present, there is no specific, rapid, and reliable method for diagnosing CFS. *See* http://www.cdc.gov/cfs/case-definition/index.html, Centers for Disease Control and Prevention, July 2012.

The search for the etiology(ies) and the pathogenic mechanisms of CFS has reviewed several hypotheses and has lived a shocking wave after the identification of XMRV (xenotropic murine leukemia virus-referred virus) as a potential candidate. *See* Papadopoulos A, et al., Nat. Rev. Endocrinol. 2012; 8: 22-32, Lombardi V, et al., Science 2009; 326: 585-589, Lo S, et al., Proc. Natl. Acad. Sci. USA 2010; 107: 15874-15879, and Wainberg M, et al., Cell Host Microbe 2011; 9: 260-262. Although, this hypothesis has been promptly ruled out, the emergence of a retrovirus in the CFS field has boosted previous research in both the virological and the immunological sides of the illness, bringing new information on the potential role of immune cells in CFS pathogenesis. *See* Shin C, et al., J. Virol. 2011; 85:7195-7202, Simmons G, et al., Science 2011; 334:814-817, Silverman R, et al., Science 2011; 334:176, Brenu E, et al., J. Transl..Med. 2011; 9: 81, Bansal A, et al., Brain Behav. Immun. 2012; 26: 24-31, and Nijs J, et al., Eur. J. Clin. Invest. 2012; 42:203-212. The link between the immune system and CFS has been explored since early 90's, and is supported by the coincidence of the onset of symptoms with viral infections, by the potential role of autoimmunity and by an undefined higher prevalence or lack of containment of several infections that are no pathogenic for the immunocompetent population. *See* Bansal, 2012, *supra,* Landay A, et al., Lancet 1991; 338:707-712, Galbraith S, et al., J. Infect. Dis. 2011; 204:1632-1640, Fluge O, et al., PLoS One 2011; 6:e26358, Fluge O, et al., BMC Neurol. 2009; 9:28, Chia J, et al., J. Clin. Pathol. 2010; 63:165-168, Chia J, et al., J. Clin. Pathol. 2008; 61:43-48, Kerr J, et al., J. Gen. Virol. 2010; 91:893-897, Koelle D, et al., Clin. Infect. Dis. 2002; 35:518-525.

Strikingly, the characterization of the immune status of CFS individuals has frequently yield contradictory results. Early work pointed to a general status of immune activation assessed by CD38 or HLA-DR expression, paralleling somehow HIV infection; however, similar levels of these markers have been described in CFS and healthy individuals. *See* Landay, 1991, *supra,* Massanella M, et al., AIDS 2010; 24: 959-968, Negredo E, et al., Clin. Infect. Dis. 2010; 50: 1300-1308, Swanink C, et al., J. Infect. Dis. 1996; 173:460-463. Quantitative or qualitative defaults in NK cells have been more consistently associated with CFS, although controversial data have been also reported and a consensus on the altered phenotype or function is still lacking. Similarly, affected B cell function, B cell mediated autoimmunity or unbalanced cytokine network have been linked to CFS again with inconclusive results. *See* Brenu, 2011, *supra,* Morrison L, et al., Clin. Exp. Immunol. 1991; 83:441-446, Buchwald D, et al., Rev. Infect. Dis. 1991; 13:S12-S18, Barker E, et al., Clin. Infect. Dis. 1994; 18 Suppl 1: S136-S141, Stewart C, et al., Cytometry B. Clin. Cytom. 2003; 53: 26-33, Robertson M, et al., Clin. Exp. Immunol. 2005; 141:326-332, Fletcher M, et al., J. Transl. Med. 2009; 7: 96, Broderick G, et al., Brain Behav. Immun. 2010; 24:1209-1217, Lombardi V, et al., In vivo 2011; 25: 307-314. At least some of these immune features described in CFS may be referred to active, poorly controlled or latent viral infections, which differently modulate immune responses and may produce both immune hyperactivation and exhaustion, as widely reported for HIV; may contribute to immunosenescence, as postulated for CMV; or may cause a general status of immune anergy, as described for measles virus. *See* Massanella, 2010, *supra,* Gress R, et al., J. Clin. Invest. 2009; 119:2884-2887, and Griffin D, et al., Immunol. Rev. 2010; 236: 176-189.

Although some markers have been reported to be correlated to CFS previously, no conclusive criteria for diagnosing CFS have been developed so far. For instance, various NK cells phenotypes and FoxP3+ expression have been disclosed as parameters useful for identifying CFS patients. *See* Brenu, 2011, *supra.* However, CFS diagnosis continues to rely at present on observing the symptoms of the presumptive CFS patient for several months. There is no method known in the art for diagnosing CFS rapidly and accurately.

### Summary of the Invention

The present invention refers to a panel of molecular markers which allows the identification of patients suffering CFS with a sensitivity and specificity that surpasses other methods known in the art. In particular, the method is based on differences observed in the T and NK cells profiles of individuals which suffer CFS. These differences make possible identifying CFS patients from the general population with a reasonable degree of accuracy.

An attempt to diagnose CFS based on criteria besides the observation of the presumptive CFS patient was disclosed in Brenu, 2011, *supra.* However, the Brenu assay has significant differences and disadvantages in comparison to the method of the invention, namely:
i) in addition to the FoxP3+ marker disclosed by Brenu, 2011, *supra,* the method of the invention is based on assaying CD56 expression in CD3+ T cells; CD5 expression and frequency of effector cells in CD8+ T cells and, alternatively to FoxP3+, Ki67 in CD4+ T cells,
ii) instead of specific markers in a particular NK cell phenotype (i.e. CD69, CD25 or NKp46 in CD56+CD16+) as in the present method, Brenu, 2011, *supra,* proposes the relative abundance of NK phenotypes (i.e. CD56BrightCD16- and CD56DimCD16+; especially CD56BrightCD16-) as indicative of a CFS patient, and
iii) although Brenu, 2011, *supra,* identifies several biomarkers as potential CFS predictors, it fails to set out a high accuracy selection method as in the present assay. *See* Example 6.

In a first aspect, the invention refers to a method for diagnosing CFS or a predisposition thereto *in vitro* which comprises:
a) determining the levels of the CD25, CD69, NKp46 and CD57 biomarkers in CD56+CD16+ NK cells in a sample obtained from a subject, and
b) comparing the levels obtained in a) with a reference value for each marker, wherein increased levels of CD69 and NKp46 and decreased levels of CD25 and CD57 with respect to the reference value for each marker is indicative that the subject suffers or is predisposed to suffering CFS.

In a second aspect, the invention refers to a kit comprising reagents for assaying the levels of CD25, CD69, NKp46 and CD57 in CD56+CD16+ NK cells.

In a third aspect, the invention refers to the use of a kit of the invention for the diagnosis of CFS or of a predisposition thereto.

In a fourth aspect, the invention refers to a reagent for inhibiting the expression or activity of at least one biomarker selected from the group consisting of CD69, NKp46 and CD5 for use in the treatment or prevention of CFS in a subject in need thereof.

In a fifth aspect, the invention refers to a reagent for inducing the expression or activity of at least one biomarker selected from the group consisting of CD25, CD57, CD56 and Ki67 for use in the treatment or prevention of CFS in a subject in need thereof.

In a sixth aspect, the invention refers to a kit comprising a reagent of the invention.

In a seventh aspect, the invention refers to the use of a kit of the invention in the treatment or prevention of CFS in a subject in need thereof.

### Brief Description of the Drawings

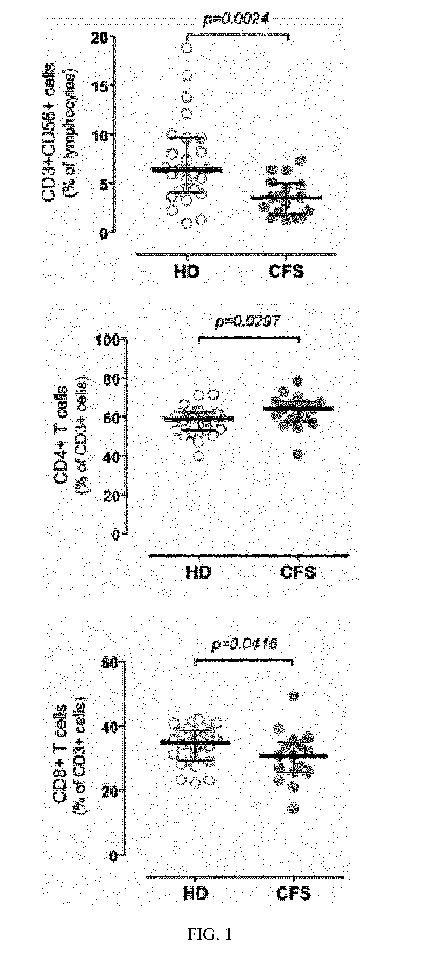

**Figure 1****.** Analysis of major lymphocyte subsets in CFS affected individuals. Fresh blood was stained with anti CD45, CD19, CD3, CD4, CD8, CD16 and CD56 antibodies. The percentage of NK (CD3―CD56+CD16+/-), B (CD19+) and T cells (CD3+) was analyzed in gated CD45+ lymphocytes. Similarly, after gating CD3+ lymphocytes the percentage of CD4+, CD8+ or CD56+ cells was analyzed. Figures show individual data from healthy donors (n=24, open dots) and SFC affected individuals (n=17, solid dots) with median values (thick lines) and interquartile ranges (bars). In all cases, p-values for nonparametric Mann-Whitney comparison are shown.

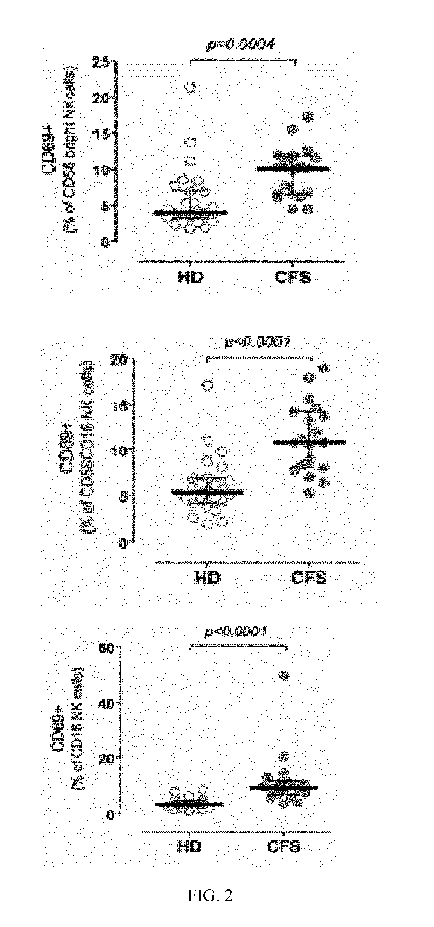

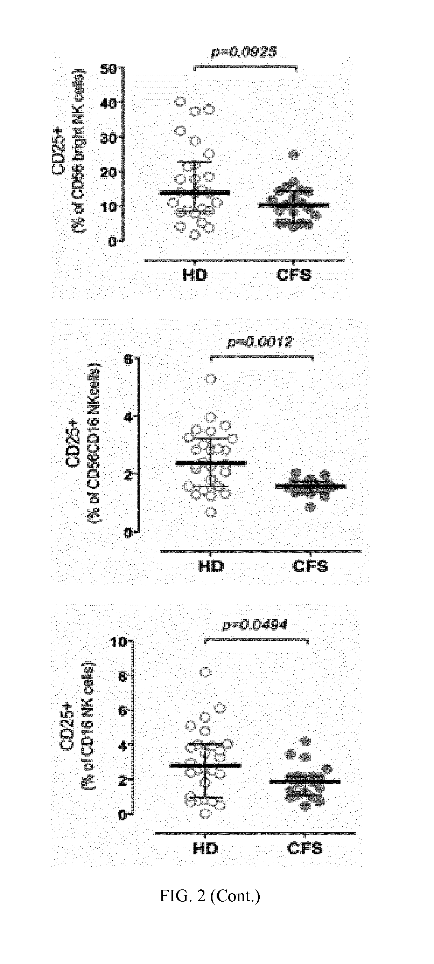

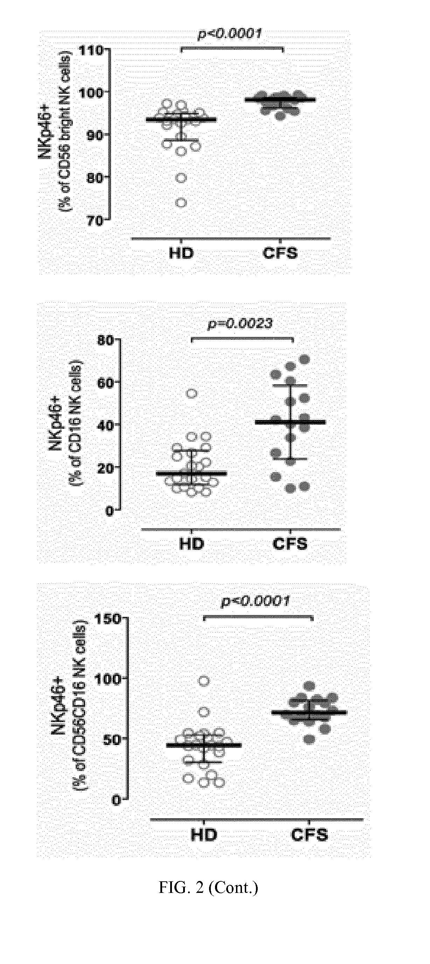

**Figure 2**. Analysis of NK cell phenotype in CFS affected individuals. Fresh blood was stained with the antibody combinations described in Table 2, lysed, washed and acquired. A. NK cells were gated according to CD16 and CD56 staining in CD56 bright (left panels), CD56+CD16+ (middle panels) or CD16+ (right panels). The expression of CD69 (upper), CD25 (middle) and NKp46 receptor is shown.

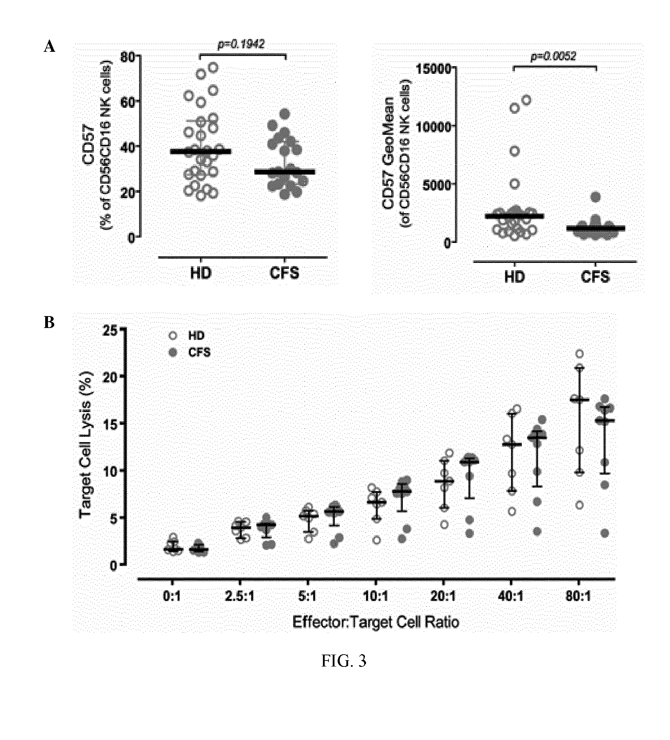

**Figure 3**. A. Double positive CD56+CD16+ NK cells were analyzed for the expression of CD57, as the percentage of positive cells (upper graph) or the Mean Fluorescence Intensity (lower graph). In all cases, data from healthy donors (n=25, open dots) and SFC affected individuals (n=19, solid dots) are shown, with median values (thick lines), interquartile ranges (bars) and p values for nonparametric Mann-Whitney comparison. B. Analysis of NK cell lytic activity. Increasing amounts of monocyte-depleted lymphocytes from healthy donors (n=7, open dots) and SFC affected individuals (n=9, solid dots) were cultured for 4 hours with 10,000 eGFP-K562 cells. The percentage of dead target cells (PI+), assumed to be the NK cell lytic activity is shown for the different ratios of effector and target cells analyzed (from 2.5:1 to 80:1). A culture of eGFP-K562 cells alone was used as a control of background levels of cell death (ratio 0:1, left part of the graph).

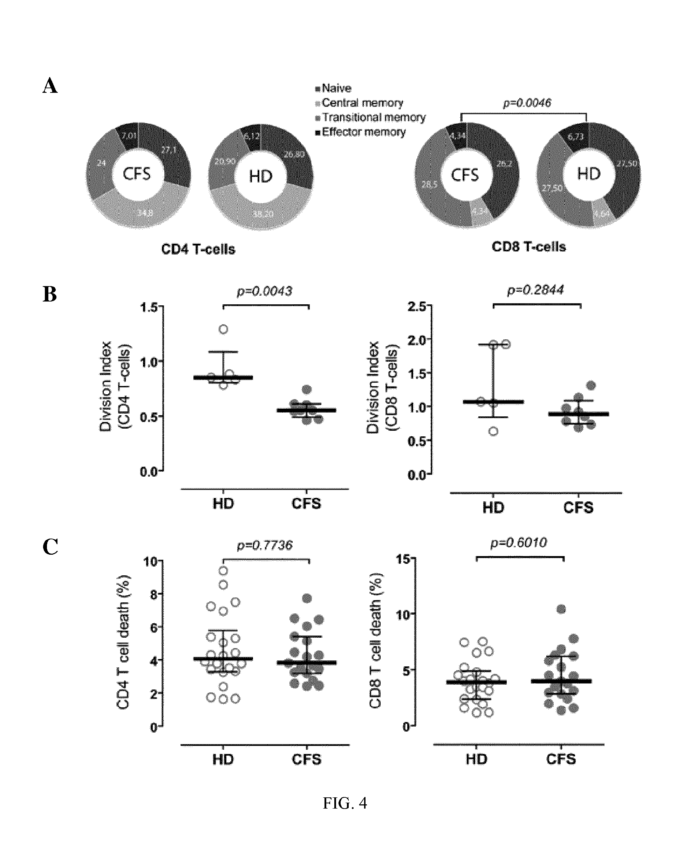

**Figure 4**. Analysis of CD4 and CD8 T cell subsets, proliferation and death in CFS affected individuals. A. Fresh blood was stained with the antibody combinations described in Table 2, lysed, washed and acquired. Different CD4 and CD8 T cell subpopulations (naive, central memory, transitional memory and effector memory) are shown as the median values for healthy donors and CFS affected individuals in circular plots. Significant differences among groups are indicated. B. In parallel, PBMC from healthy donors (n=5, open dots) and CFS affected individuals (n=8, solid dots) were stained with CFSE and cultured in the presence of a combination of PHA and IL-2 (PHA/IL-2). Data shown are division index of gated CD4 or CD8 T cells calculated from best-fit curves using FlowJo software. C. PBMC from healthy donors (n=30, open dots) and SFC affected individuals (n=19, solid dots) were also cultured for 24 h to assess spontaneous CD4 or CD8 T cell death using DIOC6 and PI staining. Results show total cell death defined by low DIOC6 fluorescence signal. In panels B and C, median values (thick lines), interquartile ranges (bars) and p-values for non-parametric Mann-Whitney comparison are shown.

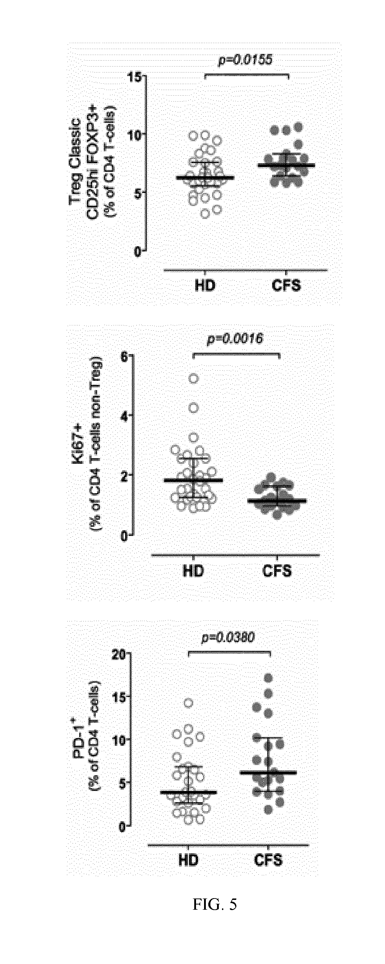

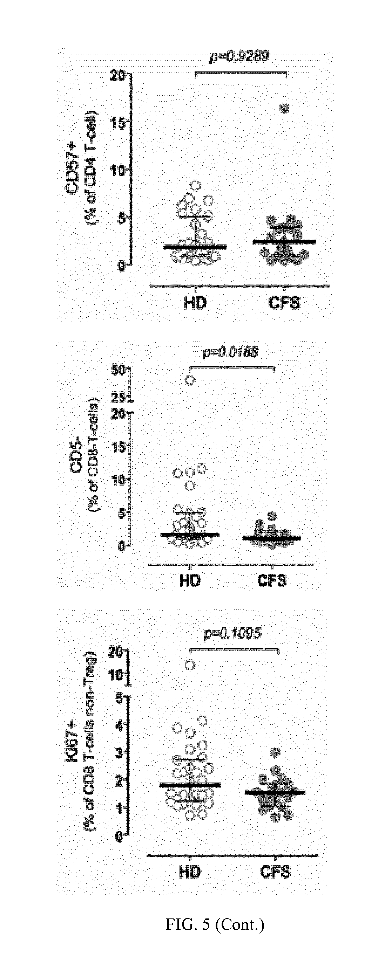

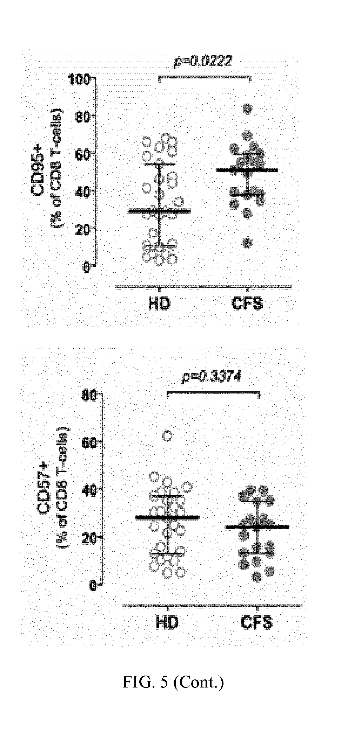

**Figure 5**. Analysis of different CD4 and CD8 T cell markers. Fresh blood from healthy donors (n=30, open dots) and SFC affected individuals (n=19, solid dots) was stained with the antibody combinations described in Table 2, lysed washed and acquired. Different CD4 (A) and CD8 T cell (B) markers are plotted. In all cases, median values (thick lines), interquartile ranges (bars) and p values for nonparametric Mann-Whitney comparison are shown.

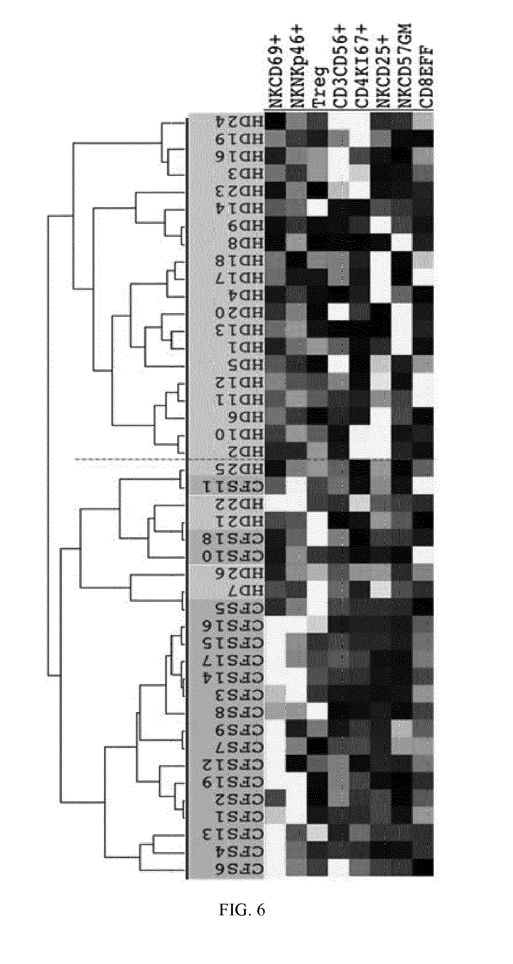

**Figure 6**. Clustering CFS individuals according to NK and T cell phenotypic markers. A subset of 19 CFS (red labels) and 25 control individuals was analyzed. The figure shows normalized centered color coded data, positive values, above median in yellow and negative values, below median in blue. Two groups of CFS and control individuals were clearly differentiated, while a heterogeneous subgroup was clustered with CFS individuals.

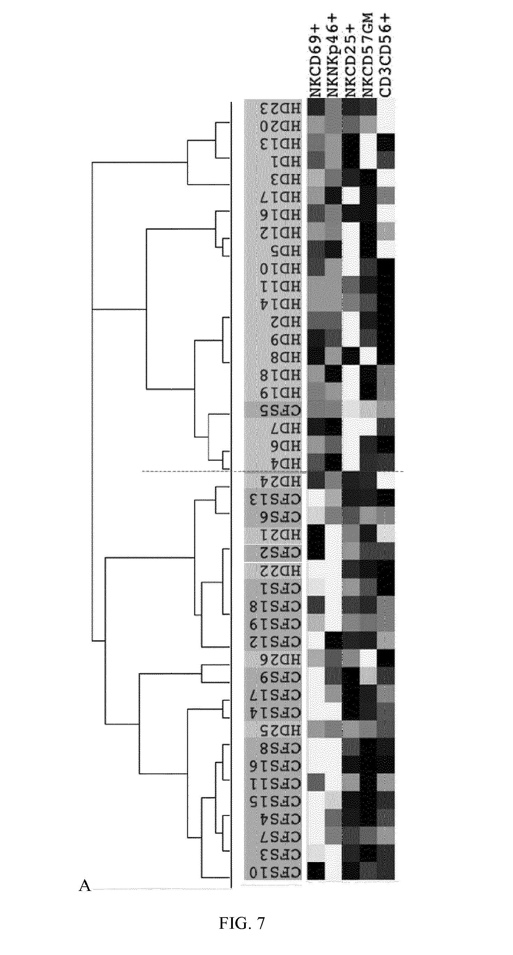

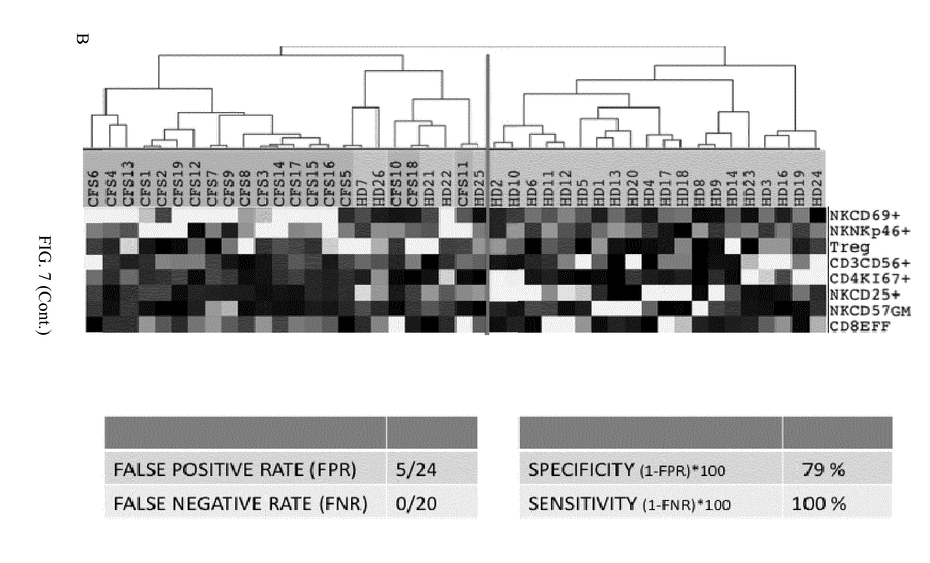

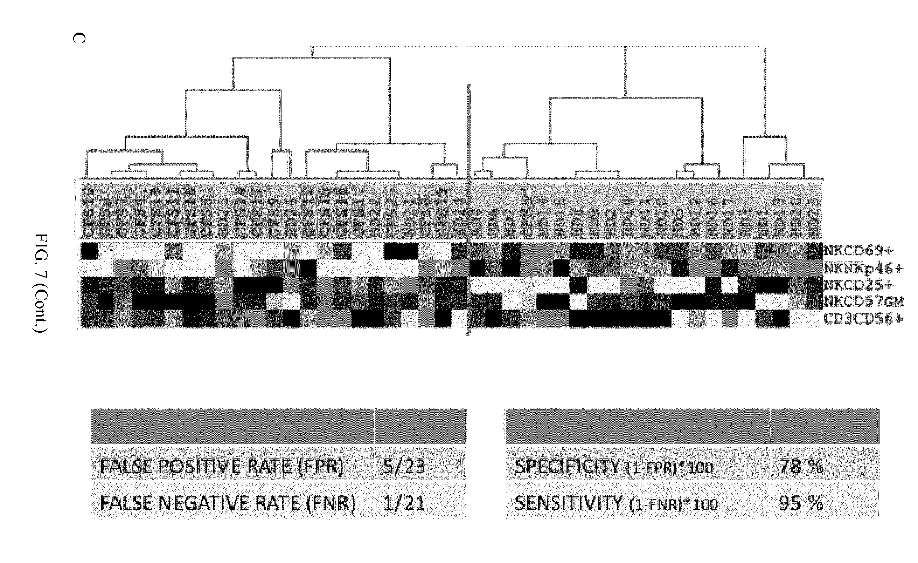

**Figure 7**. Predictive value of NK cell markers. Association between observed T and NK cells phenotypic alterations and CFS. A cluster analysis taking into account marker selectivity yielded promising good CFS predictive capacity (A). The combination of the first and third biomarker panels exhibited the best resolution in classifying CFS and healthy individuals. This combination showed a high sensitivity (100%) but a moderate false positive rate (5/24, specificity 79%) (B). A more restrictive choice of parameters, based on the first biomarker panel exclusively showed similar false positive rates (5/23, specificity 78%) but lower sensitivity (95%) in the detection of CFS cases (C).

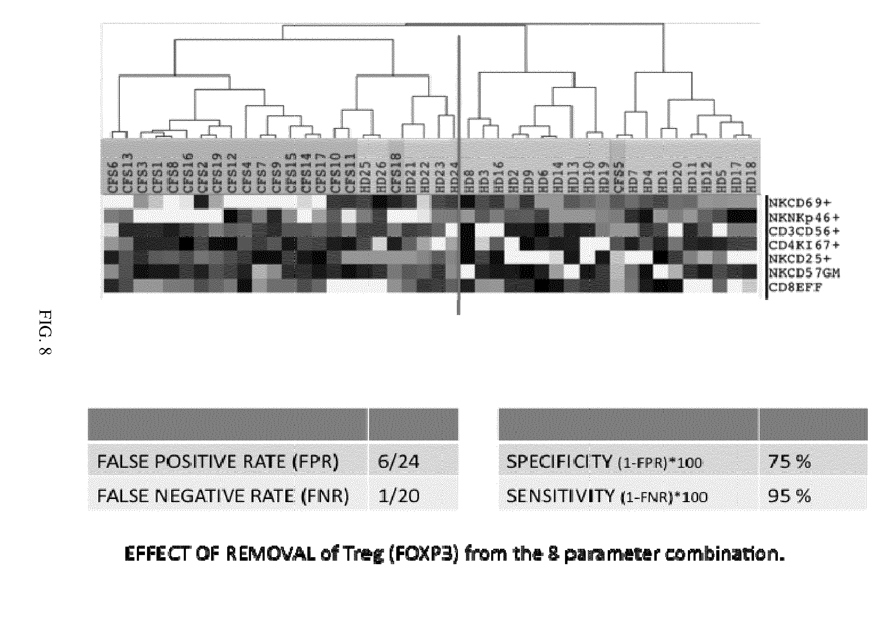

**Figure 8****.** Analysis of the specific predictive weight of the FoxP3+ marker in the joint first and third biomarker panel shown in Figure 6. A modest loss of specificity and sensitivity was observed, suggesting that the FoxP3+ marker is not a necessary parameter in the cluster.

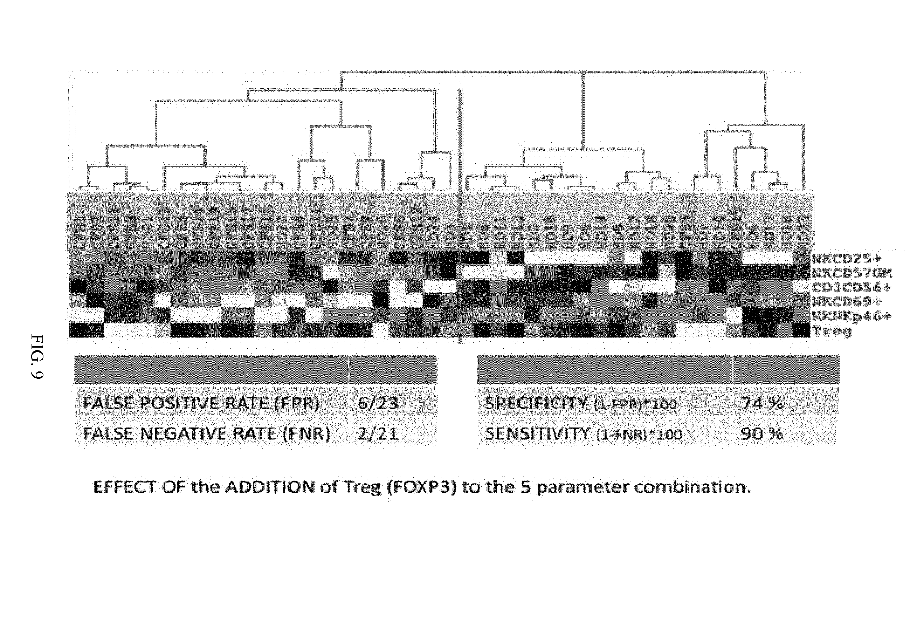

**Figure 9**. Analysis of the specific predictive contribution of the FoxP3+ marker to the first biomarker panel. The FoxP3+ maker did not improve the specificity and sensitivity of the first biomarker panel. This result suggests that the FoxP3+ marker alone did not increase the clustering of NK parameters and that; consequently, other T cell parameters, such as the elements of the second biomarker panel, are required for optimal clustering.

### Detailed Description of the Invention

The present invention refers to a method for diagnosing CFS based on differences observed in the T and NK cells profiles of individuals which suffer CFS. These subjects showed increased levels of T regulatory cells CD25⁺/FoxP3⁺ CD4 T cells, and low proliferative responses *in vitro* or *in vivo* compared to healthy individuals. Moreover, CD8 T cells showed low activation and reduced effector memory cells in the CFS group. In addition, even though both groups showed similar NK cell lytic ability *in vitro,* CFS patients displayed higher expression of NKp46 and CD69, but lower expression of CD25 in all NK subsets. These differences make possible identifying CFS patients from the general population with a reasonable degree of accuracy.

### 1. Definitions of general terms and expressions

The term "AIDS", as used herein, refers to the symptomatic phase of HIV infection, and includes both Acquired Immune Deficiency Syndrome (commonly known as AIDS) and "ARC," or AIDS-Related Complex. *See* Adler M, et al., Brit. Med. J. 1987; 294: 1145-1147. The immunological and clinical manifestations of AIDS are known in the art and include, for example, opportunistic infections and cancers resulting from immune deficiency.

The term "B cell", as used herein, refers to any member of a diverse population of morphologically similar cell types that develop in the bone marrow and that mediate the humoral immune response of the adaptive immune system. B cells are characterized by the presence of a B cell receptor able to bind specifically an antigen. Their principal functions are to make antibodies against antigens, perform the role of antigen-presenting cells (APCs) and eventually develop into memory B cells after activation by antigen interaction. *See* Alberts B, et al., "Molecular Biology of the Cell" (Garland Publishing Inc., New York, NY, US, 2008, pp. 1363-1391).

The term "CD3+ T cells", as used herein, refers to a type of cells that express the CD3 marker. "CD3", as used herein, refers to a cluster of differentiation 3, a protein complex composed of four distinct chains. In mammals, the complex contains a CD3γ chain, a CD3δ chain, and two CD3ε chains. These chains associate with a molecule known as the T cell receptor (TCR) and the ζ-chain to generate an activation signal in T lymphocytes. The TCR, ζ-chain, and CD3 molecules together comprise the TCR complex. The complete protein sequence for human CD3 has the UniProt accession number P07766 (June 18^{th}, 2012).

The term "CD4+ T cells", as used herein, refers to a type of T cell that expresses the CD4 marker. Said CD4+ T cells are generally treated as having a pre-defined role as helper T cells within the immune system. "CD4", as used herein, refers to a cluster of differentiation 4, a glycoprotein expressed on the surface of T helper cells, monocytes, macrophages, and dendritic cells. CD4 is a co-receptor that assists the T cell receptor (TCR) with an antigen-presenting cell. Using its portion that resides inside the T cell, CD4 amplifies the signal generated by the TCR by recruiting an enzyme, known as the tyrosine kinase lck, which is essential for activating many molecules involved in the signaling cascade of an activated T cell. The complete protein sequence for human CD4 has the UniProt accession number P01730 (June 18^{th}, 2012).

The term "CD5", as used herein, refers to a cluster of differentiation found on a subset of IgM-secreting B cells called B-1 cells, and also on T cells wherein CD5 is upregulated upon strong activation. The complete protein sequence for human CD5 has the UniProt accession number P06127 (June 18^{th}, 2012).

The term "CD8+ T cells", as used herein, refers to a type of T cell that expresses the CD8 marker. "CD8", as used herein, refers to cluster of differentiation 8, a transmembrane glycoprotein that serves as a co-receptor for the T cell receptor (TCR) expressed in the cytotoxic T cells implicated in the rejection of transplants and the destruction of tumor and virally infected cells. The complete protein sequence for human CD8 has the UniProt accession number P10966 (June 18^{th}, 2012).

The term "CD25", as used herein, refers to the alpha chain of the IL-2 receptor, a type I transmembrane protein present on activated T cells, activated B cells, activated NK cells, some thymocytes, myeloid precursors, and oligodendrocytes that associates with CD 122 to form a heterodimer that can act as a high-affinity receptor for IL-2. The complete protein sequence for human CD25 has the UniProt accession number P01589 (June 18^{th}, 2012).

The term "CD56+CD16+ NK cells", as used herein, refers to a type of NK cell that expresses the CD56 and CD16 markers. "CD56", as used herein, refers to a 175-185 kD homophilic binding glycoprotein expressed on the surface of neurons, glia, skeletal muscle and natural killer cells. The alternative name of CD56 is N-CAM (i.e. neural cell adhesion molecule). The complete protein sequence for human CD56 has the UniProt accession number P13591 (June 18^{th}, 2012). "CD16", as used herein, refers to a glycosylphosphatidylinositol-anchored protein which plays a significant role in the clearance of immune complexes, antibody-dependent cellular cytotoxicity of NK cells, phagocytosis and antigen presentation. The complete protein sequence for human CD16 has the UniProt accession number Q9ULV2 (June 18^{th}, 2012). The isolation of CD56+CD16+ NK cells can be performed by methods known in the art, such as FACS (fluorescent-activated cell sorting) or MACS (magnetic-activated cell sorting). There are many kits available commercially for isolating this type of NK cells (e.g. MACS@ CD56+CD16+ NK isolation kit, catalogue number 130-092-660, Miltenyi Biotec GmbH, Bergisch Gladbach, DE).

The term "CD57", also called HNK1 (human natural killer-1) or LEU7, as used herein, refers to a membrane protein present on NK cells, some T cells, a few B cells, and monocytes of unknown function and which is expressed in large granular lymphocyte leukemias. The complete protein sequence for human CD57 has the UniProt accession number Q9P2W7 (June 18^{th}, 2012).

The term "CD69", as used herein, refers to a molecule, which appears to be the earliest inducible cell surface glycoprotein acquired during lymphoid activation, is involved in lymphocyte proliferation and functions as a signal-transmitting receptor in lymphocytes, including NK cells, and platelets. The complete protein sequence for human CD69 has the UniProt accession number Q07108 (June 18^{th}, 2012).

The term "Chronic Fatigue Syndrome" or "CFS", as used interchangeably herein, refer to a debilitating and complex disorder characterized by intense fatigue that is not improved by bed rest and that may be worsened by physical activity or mental exertion. Individuals with CFS often function at a substantially lower level of activity than they were capable of before they became ill. The cause or causes of CFS have not been identified, and no specific diagnostic tests are available. Presently, a CFS diagnosis is based on three criteria: i) the individual has had severe chronic fatigue for 6 or more consecutive months that is not due to ongoing exertion or other medical conditions associated with fatigue, ii) the fatigue significantly interferes with daily activities and work and iii) the individual concurrently has 4 or more of the following 8 symptoms: a) post-exertion malaise lasting more than 24 hours, b) unrefreshing sleep, c) significant impairment of short-term memory or concentration, d) muscle pain, e) pain in the joints without swelling or redness, f) headaches of a new type, pattern, or severity, g) tender lymph nodes in the neck or armpit, or h) a sore throat that is frequent or recurring. These symptoms should have persisted or recurred during 6 or more consecutive months of illness and they cannot have first appeared before the fatigue. *See* http://www.cdc.gov/cfs/case-definition/index.html, Centers for Disease Control and Prevention, July 2012.

The term "comprising" or "comprises", as used herein, discloses also "consisting of" according to the generally accepted patent practice.

The expression "determining the level of a biomarker", as used herein, refers to assaying the expression level of a biomarker or the number of cells exhibiting a given biomarker on their surface (i.e. a cell surface marker). The level of expression refers to the level of mRNA, the level of protein or the number of cells carrying a given biomarker on their surface.

The term "effector cells" as used herein, refers to a subtype of CD8+ T cells characterized in that they are CD45RA⁻/⁺ CCR7⁻CD28⁻CD27⁻ cells.

The term "FoxP3", as used herein, refers to forkhead box P3 also known as scurfin, a protein involved in immune system responses. FoxP3 appears to function as a master regulator in the development and function of regulatory T cells (Treg). The complete protein sequence for human FoxP3 has the UniProt accession number B7ZLG1 (June 18^{th}, 2012).

The expression "functionally equivalent variant of a marker", as used herein, refers to: i) variants of the marker in which one or more of the amino acid residues are substituted by a conserved or non-conserved amino acid residue (preferably a conserved amino acid residue), wherein such substituted amino acid residue may or may not be one encoded by the genetic code, or ii) variants comprising an insertion or a deletion of one or more amino acids and having the same function as the marker.

The term "HIV", as used herein, include HIV-1 and HIV-2 and SIV. "HIV- 1" means the human immunodeficiency virus typo-1. HIV-1 includes, but is not limited to, extracellular virus particles and the forms of HIV-1 associated with HIV-1 infected cells. The HIV-1 virus may represent any of the known major subtypes (Classes A, B, C, D E, F, G and H) or outlying subtype (Group O) including laboratory strains and primary isolates. "HIV-2" means the human immunodeficiency virus type-2. HIV-2 includes, but is not limited to, extracellular virus particles and the forms of HIV-2 associated with HIV-2 infected cells. The term "SIV" refers to simian immunodeficiency virus which is an HIV-like virus that infects monkeys, chimpanzees, and other nonhuman primates. SIV includes, but is not limited to, extracellular virus particles and the forms of SIV associated with SIV infected cells.

The term "Ki67", also known as MKI67, as used herein, refers to a nuclear protein associated with cellular proliferation. The expression of this antigen occurs preferentially during the G1, S, G2 and M phases of the cellular cycle. The antigen cannot be detected in cells during the G0 phase. The complete protein sequence for human Ki-67 has the UniProt accession number P46013 (June 18^{th}, 2012).

The term "kit", as used herein, refers to a product containing the different reagents necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes.

The expression "method for the diagnosis", as used herein, refers to a process that consists essentially of the steps of the method of the invention or that may include additional steps. "Diagnosing", as used herein, refers to evaluating the probability according to which a subject suffers from a disease. The method may be carried out *in vitro* (i.e. it is not performed over the human or animal body).

The term "NK cell", as used herein, refers to a type of cytotoxic lymphocyte critical to the innate immune system. NK cells provide rapid responses to virally infected cells and respond to tumor formation, acting at around 3 days after infection. Typically immune cells detect HLA presented on infected cell surfaces, triggering cytokine release causing lysis or apoptosis. NK cells are unique, however, as they have the ability to recognize stressed cells in the absence of antibodies and HLA, allowing for a much faster immune reaction. NK cells are defined as large granular lymphocytes and constitute the third kind of cells differentiated from the common lymphoid progenitor generating B and T lymphocytes. NK cells are known to differentiate and mature in the bone marrow, lymph node, spleen, tonsils and thymus where they then enter into circulation. NK cells express usually the surface markers CD16 (FcγRIII) and CD56 in humans, and the NK1.1 or NK1.2 markers in C57BL/6 mice. Up to 80% of human NK cells also express the CD8 marker. *See* Alberts, 2008, *supra,* pp. 1285-1286, 1461-1462.

The term "NKp46" or "natural killer cell p46-referd protein", as used herein, refers to a protein of 46 kDa expressed on resting and activated NK-cells but not on T cells or B cells involved in the mechanism of NK cell activation during natural cytotoxicity. The complete protein sequence for human NKp46 has the UniProt accession number 076036 (June 18^{th}, 2012).

The term "predisposition", as used herein, refers to the likelihood that a subject, who has not still developed a disease or shown any of the symptoms of a disease, may develop said disease in the future.

The terms "prevent," "preventing," and "prevention", as used herein, refer to inhibiting the inception or decreasing the occurrence of a disease in a subject. Prevention may be complete (e.g. the total absence of pathological cells in a subject) or partial. Prevention also refers to a reduced susceptibility to a clinical condition.

The expression "reagent which allows determining the levels of a marker", as used herein, refers to a compound or set of compounds that allows determining the level of expression of a marker.

The expression "reagent for inducing the expression or activity" refers to any molecule capable of increasing the expression of a gene (e.g. by prompting its transcription or its mRNA translation) or increasing the activity of a protein. The induction can be complete or partial.

The expression "reagent for inhibiting the expression or activity" refers to any molecule capable of arresting the expression of a gene (e.g. by interrupting its transcription or blocking its mRNA translation) or arresting the activity of a protein. The inhibition can be complete or partial.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The term "sample", as used herein, refers to any sample collected from a subject, in which the markers of the methods of the invention can be measured. Suitable samples for use in the present invention include any body fluid and, in particular, blood, serum, plasma, lymph, saliva, peripheral blood cells or tissue cells serum, semen, sputum, cephalorachidian liquid (CRL), tears, mucus, sweat, milk, or brain extracts. The bodily tissue may comprise thymus, lymph node, spleen, bone marrow or tonsil tissue. The biological test sample may comprise at least one isolated lymphocyte or at least one natural killer cell.

The term "subject", as used herein, refers to an individual, plant or animal, such as a human beings, a non-human primate (e.g. chimpanzees and other apes and monkey species), a farm animal (e.g. birds, fish, cattle, sheep, pigs, goats and horses), a domestic mammal (e.g. dogs and cats), or a laboratory animal (e.g. rodents, such as mice, rats and guinea pigs. The term does not denote a particular age or sex. The term "subject" encompasses an embryo and a fetus.

The term "T cell", as used herein, refers to any member of a diverse population of morphologically similar lymphocytes types that develop in the thymus and that mediate the cellular immune response of the adaptive immune system. They are characterized by the presence of a T cell receptor on the cell surface. There are several subsets of T cells, each with a distinct function (i.e. helper, memory, regulatory, natural killer). *See* Alberts, 2008, *supra,* pp. 1364-1374, 1392-1409.

The term "Treg" or "regulatory T cell", as used herein, refers to a T cell that expresses the CD4 or CD25 marker at least and which is capable of reducing or suppressing the activity of a T cell. This term includes T cells producing low levels of IL-2, IL-4, IL-5, and IL-1, and which suppress the activation of the immune system. Regulatory T cells suppress actively the proliferation and cytokine production of TH₁, TH₂, or naive T cells which have been stimulated in culture with an activating signal (e.g. antigen and antigen presenting cells or with a signal that mimics antigen in the context of HLA, such as, for instance, an anti-CD3 antibody plus an anti-CD28 antibody). Treg cells may express the FoxP3 marker.

The term "treat" or "treatment", as used herein, refers to the administration of a compound of the invention or of a composition or medicament containing it to control the progression of a disease after its clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival, compared with the expected survival if treatment was not applied.

### 2. Methods for diagnosing or for determining predisposition to CFS

In a first aspect, the invention refers to a method for diagnosing CFS or a predisposition thereto *in vitro* which comprises:
a) determining the levels of the CD25, CD69, NKp46 and CD57 biomarkers in CD56+CD16+ NK cells (hereinafter referred to "first biomarker panel") in a sample obtained from a subject, and
b) comparing the levels obtained in a) with a reference value for each marker, wherein increased levels of CD69 and NKp46 and decreased levels of CD25 and CD57 with respect to the reference value for each marker is indicative that the subject suffers or is predisposed to suffering CFS. Preferably, the sample is a body fluid. More preferably, the body fluid is blood.

The methods for determining the level of the biomarkers according to the present invention can be based on assaying the level of expression of RNA or protein in the sample as a whole, in cells of the sample or in the non-cellular fraction of the sample. Methods for assaying mRNA levels are known in the art (e.g. real-time PCR (rtPCR), Northern blotting, nanostring and microarray technologies).

Virtually any conventional method can be used within the frame of the invention to detect and quantify the levels of biomarkers. By way of a non-limiting illustration, the expression levels can be determined by means of antibodies with the capacity for binding specifically to the assayed protein (or to fragments thereof containing the antigenic determinants) and subsequent quantification of the resulting antigen-antibody complexes. The antibodies can be monoclonal, polyclonal or fragment thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known test that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, immunofluorescence, techniques based on the use of biochips or protein microarrays including specific antibodies or assays based on the colloidal precipitation in formats such as reagent strips. Other forms of detecting and quantifying protein include, for instance, affinity chromatography techniques or ligand-binding assays.

In a preferred embodiment of the invention, the determination of the levels of biomarkers is performed by quantitative immunofluorescence. Immunofluorescence (IF) is a technique based on fluorescent microscopy utilized primarily for testing biological samples. This technique uses the specificity of antibodies to their antigen to target fluorescent dyes to particular biomolecule targets within a cell, thus allowing the visualization of the distribution of the target molecule through the sample. IF can be used on tissue sections, cultured cell lines, or individual cells, and may be used to analyze the distribution of proteins, glycans, and small biological and non-biological molecules. In addition, IF could also be used in combination with other, non-antibody methods of fluorescent staining, such as, for example, DAPI to label DNA. Several microscope designs can be used for analysis of IF samples, such as the epifluorescence microscope and the confocal microscope. Various super-resolution microscope designs that are capable of much higher resolution may also be used.

Fluorescence labels are contemplated herein. The labels may be fluorochromes or other measurable reagents. Selection of one or more labels for use will depend on the lasers used to excite the fluorochromes or cytochromes and the available detectors. An example of a label includes, but is not limited to, Blue (488 nm); Green (usually labeled FL1): FITC₁ Alexa Fluor 488, GFP, CFSE, CFDA-SE, DyLight 488; Orange (usually FL2): PE, Pl; Red channel (usually FL3): PerCP, PerCP- Cy5.5, PE-Alexa Fluor 700, PE-Cy5 (TRI-COLOR), PE-Cy5.5.; Infra-red (usually FL4; not provided by all FACS machines as standard): PE-Alexa Fluor 750, PE-Cy7; Red diode laser (635 nm); APC; APC-Cy7, APC-eFluor 780; Alexa Fluor 700; Cy5; Draq-5; Violet laser (405 nm); Pacific Orange; Amine Aqua; Pacific Blue; DAPI; Alexa Fluor 405; eFluor 450; eFluor 605 Nanocrystals; eFluor 625 Nanocrystals and eFluor 650 Nanocrystals.

In a more preferred embodiment, the level of the biomarkers can be determined, for example, by means of flow cytometry using conventional methods and apparatuses. For instance, a BD LSR II Flow Cytometer (BD Biosciences Corp., Franklin Lakes, NJ, US) with commercially available antibodies and following protocols known in the art may be employed. Thus, cells emitting a signal for a specific cell surface marker more intense than the background noise can be selected. The background signal is defined as the signal intensity given by a non-specific antibody of the same isotype as the specific antibody used to detect each surface marker in the conventional FACS analysis. In order for a marker to be considered positive, the observed specific signal must be 20%, preferably, 30%, 40%, 50%, 60%, 70%, 80%, 90%, 500%, 1000%, 5000%, 10000% or above more intense than the background signal using conventional methods and apparatuses (e.g. by using a FACSCalibur flow cytometer (BD Biosciences Corp., Franklin Lakes, NJ, US) and commercially available antibodies).

In addition, the expression level can also be assessed by quantifying the expression of any functionally equivalent variant of a biomarker. Preferably, a variant will have a similarity of at least 50%, at least 60%, at least 70%, at least 80%, at least 90%, at least 91%, at least 92%, at least 93%, at least 94%, at least 95%, at least 96%, at least 97%, at least 98%, or at least 99% of the amino acid sequence of any biomarker isoform of the invention. The degree of similarity between a variant and biomarker can be determined by using algorithms and computer processes widely known in the art. Examples of algorithms suitable for determining percent sequence identity and sequence similarity are the BLAST and BLAST 2.0 algorithms. See Altschul S, et al., Nuc. Acids Res. 1977; 25:3389-3402 and Altschul S, et al., J. Mol. Biol. 1990; 215:403-410. The BLAST and BLAST 2.0 programs can be used to determine percent sequence identity for the nucleic acids and proteins of the invention. Software for performing BLAST analyses is publicly available through the National Center for Biotechnology Information. *See* http://blast.ncbi.nlm.nih.gov/blast.cgi, July 2012.

In one embodiment, the level of a biomarker is determined as the ratio of cells expressing said marker within a given cell population. In another embodiment, the level of a biomarker is determined as the mean fluorescence intensity within a given cell population.

In the method according to the present invention, the level of CD25 is determined as the percentage of CD25+ cells within the NK cell population (CD56+CD16+ cells). In another embodiment, the level of CD69 is determined as the percentage of CD69+ cells within the NK cell population (CD56+CD16+ cells). In one embodiment, the level of NKp46+ is determined as the percentage of NKp46+ cells within the NK cell population (CD56+CD16+ cells). In another embodiment, the level of CD57 is determined as mean fluorescence intensity obtained using a specific anti-CD57 antibody in the NK cell population (CD56+CD16+ cells).

In a preferred embodiment, the levels of CD25, CD69, NKp46 and CD57 are determined in an enriched NK cell population. A cell population enriched in NK cells can be obtained by using any one or more isolation techniques known in the art. NK cells may be enriched by negative isolation or positive isolation. Negative isolation may include antibodies binding to cells that are not of interest such as human hematopoietic cells that express the following surface antigens: CD3 as expressed on thymocytes and T cells; CD4 as expressed on thymocytes, helper and inflammatory T cells, monocytes and macrophages; CD 19 as expressed on B cells; CD36 as expressed on platelets and monocytes; CD66b as expressed on granulocytes which includes neutrophils, eosinophils and basophils; and glycophorin A which is expressed on RBCs. The negative isolation technique may include methodology already published for assaying antibodies (e.g. Rosettesep® NK enrichment cocktail, stemCell Technologies Inc., Vancouver, BC, CA). Non-limiting examples of other isolation techniques which may be negative, positive or a combination thereof would include magnetic-based techniques or affinity chromatography. Affinity chromatography may be in the form of column chromatography, immunoaffinity, immobilized metal ion affinity chromatography, recombinant protein technology and lectin-based techniques.

One non-limiting example of a magnetic-based technique is the magnetic bead technique. The beads may be coated with one or more entities that allow for specific binding to NK cells. The surface of the beads may be coated with antibodies that are directed to CD56 and CD16. Alternatively, the surface of the beads may be coated with antibodies that have a specific affinity to cell surface antigens on human hematopoietic cells that are not present on NK cells such as, for example, CD3 on thymocytes and T cells; CD4 on thymocytes, helper and inflammatory T cells, monocytes and macrophages; CD19 on B cells; CD36 on platelets and monocytes; and CD66b on granulocytes which includes neutrophils, eosinophils and basophils. The beads may also comprise glycophorin A which is directed to red blood cells. As contemplated herein, tag(s) may be used in any one or more of the above described techniques or any technique(s) that achieves NK enrichment. As a non-limiting example, a tag may be a peptide-based tag, immuno-based tag, or fluorescent tag.

The performance of the method of the invention with the first biomarker panel has a 95% specificity towards CFS patients with a false positive rate of 22%. However, the first biomarker panel of the invention can be combined with additional T cell phenotypic biomarkers to yield a method with much improved resolution (*p*= 3.3x10⁻⁸). Thus, in a preferred embodiment, the method of the invention comprises further determining the value of at least one parameter selected from the group consisting of:
i) the levels CD56 in CD3+ T cells,
ii) the frequency of Tregs in CD4+ T cells
iii) the level of Ki67 in CD4+ T cells,
iv) the level of CD5 in CD8+ T cells, and
v) the frequency of effector cells in CD8+ T cells, (hereinafter referred to "second biomarker panel") in a sample from said subject, and comparing the values obtained with reference values for each biomarker, wherein increased levels of CD5, increased frequency of Tregs in CD4+ cells, decreased levels in CD56, decreased levels of Ki67 or decreased frequency of effector cells with respect to the reference value for each biomarker is indicative that the subject suffers or is predisposed to CFS.

In one embodiment, the level of CD56 is determined as the percentage of CD56+ cells in the T cell population (CD3+ cells). In one embodiment, the frequency of Tregs in CD4+ T cells is determined as percentage of CD25 bright/FoxP3+ cells in the population of CD4 T cells. In one embodiment, the level of Ki67 is determined as the percentage of Ki67+ cells in the population of CD4+ T cells. In one embodiment, the level of CD5 is determined as the percentage of CD5+ cells in the CD8+ T cell population. In one embodiment, the frequency of effector cells is determined as the percentage of effector cells in the CD8+ T cell population. The isolation of CD3+ cells, CD4+ or CD8+ T cells can be performed by using methods known in the art, such as, for example, FACS (fluorescent-activated cell sorting) or MACS (magnetic-activated cell sorting). Commercial kits such as EasySep® Human CD3 Positive Selection Kit (StemCell Technologies Inc., Vancouver, BC, CA), the CD4+ T Cell Isolation Kit II and CD8+ T Cell Isolation Kit (Miltenyi Biotec GmbH, Bergisch Gladbach, DE) may be employed for the isolation of CD3+, CD4+ and CD8+ T cells, respectively.

In a preferred embodiment the method of the invention comprises determining the levels of the first biomarker panel as well as a subset of markers formed by a combination of any two parameters, any three parameters, any four parameters, or the five parameters of the second biomarker panel.

In a preferred embodiment, the method of the invention comprises determining the levels of the first biomarker panel and the value of a combination of any two parameters of the second biomarker panel selected from the group consisting of the levels of CD56 in CD3+ T cells and the frequency of Tregs in CD4+ T cells; the levels of CD56 in CD3+ T cells and the level of Ki67 in CD4+ T cells; the levels of CD56 in CD3+ T cells and the level of CD5 in CD8+ T cells; the levels of CD56 in CD3+ T cells and the frequency of effector cells in CD8+ T cells; the frequency of Tregs in CD4+ T cells and the level of Ki67 in CD4+ T cells; the frequency of Tregs in CD4+ T cells and the level of CD5 in CD8+ T cells; the frequency of Tregs in CD4+ T cells and the frequency of effector cells in CD8+ T cells; the level of Ki67 in CD4+ T cells and the level of CD5 in CD8+ T cells; the level of Ki67 in CD4+ T cells and the frequency of effector cells in CD8+ T cells, the level of CD5 in CD8+ T cells and the frequency of effector cells in CD8+ T cells.

In another preferred embodiment, the method of the invention comprises determining the levels of the first biomarker panel and the value of a combination of any three parameters of the second biomarker panel selected from the group consisting of the levels of CD56 in CD3+ T cells, the frequency of Tregs in CD4+ T cells and the level of Ki67 in CD4+ T cells; the levels of CD56 in CD3+ T cells, the frequency of Tregs in CD4+ T cells and the level of CD5 in CD8+ T cells; the levels of CD56 in CD3+ T cells, the frequency of Tregs in CD4+ T cells and the frequency of effector cells in CD8+ T cells; the levels of CD56 in CD3+ T cells, the level of Ki67 in CD4+ T cells and the level of CD5 in CD8+ T cells; the levels of CD56 in CD3+ T cells, the level of Ki67 in CD4+ T cells and the frequency of effector cells in CD8+ T cells; the levels of CD56 in CD3+ T cells, the level of CD5 in CD8+ T cells and the frequency of effector cells in CD8+ T cells; the frequency of Tregs in CD4+ T cells, the level of Ki67 in CD4+ T cells and the level of CD5 in CD8+ T cells; the frequency of Tregs in CD4+ T cells, the level of Ki67 in CD4+ T cells and the frequency of effector cells in CD8+ T cells; the frequency of Tregs in CD4+ T cells, the level of CD5 in CD8+ T cells and the frequency of effector cells in CD8+ T cells; the level of Ki67 in CD4+ T cells, the level of CD5 in CD8+ T cells and the frequency of effector cells in CD8+ T cells.

In another preferred embodiment, the method of the invention comprises determining the levels of the first biomarker panel and the value of a combination of any four parameters selected from the group consisting of the levels of CD56 in CD3+ T cells, the frequency of Tregs in CD4+ T cells, the level of Ki67 in CD4+ T cells and the level of CD5 in CD8+ T cells; the levels of CD56 in CD3+ T cells, the frequency of Tregs in CD4+ T cells, the level of Ki67 in CD4+ T cells and the frequency of effector cells in CD8+ T cells; the levels of CD56 in CD3+ T cells, the frequency of Tregs in CD4+ T cells, the level of CD5 in CD8+ T cells and the frequency of effector cells in CD8+ T cells; the levels of CD56 in CD3+ T cells, the level of Ki67 in CD4+ T cells, the level of CD5 in CD8+ T cells and the frequency of effector cells in CD8+ T cells; the frequency of Tregs in CD4+ T cells, the level of Ki67 in CD4+ T cells, the level of CD5 in CD8+ T cells and the frequency of effector cells in CD8+ T cells. In another preferred embodiment, the method of the invention comprises determining the levels of the first biomarker panel and the value of a combination of the five parameters, particularly the levels of CD56 in CD3+ T cells, the frequency of Tregs in CD4+ T cells, the level of Ki67 in CD4+ T cells, the level of CD5 in CD8+ T cells and the frequency of effector cells in CD8+ T cells.

In a preferred embodiment, the method of the invention comprises determining: i) the levels of the markers of the first biomarker panel and ii) the levels of CD56 in CD3+ T cells, the frequency of Tregs in CD4+ cells, the levels of Ki67 in CD4+ cells and the frequency of effector cells in CD8+ T cells (hereinafter referred to "third biomarker panel"), wherein decreased levels in CD56, increased frequency of Tregs in CD4+ cells, decreased levels of Ki67 and decreased frequency of effector cells concerning their respective biomarker reference values is indicative that the subject suffers or is predisposed to CFS. This particular embodiment of the method of the invention has a 100% specificity towards CFS patients and a relatively low false positive rate (20%).

In another embodiment, the method of the invention comprises determining: i) the levels of the markers of the first biomarker panel and ii) 3 of the 4 markers of the third biomarker panel. Thus, in this embodiment, the method comprising determining the levels of CD25, CD69, NKp46 and CD57 in CD56+CD16+ NK cells, the levels of CD56 in CD3+ T cells, the frequency of Tregs in CD4+ cells, the levels of Ki67 in CD4+ cells and the frequency of effector cells in CD8+ T cells are determined wherein increased levels of CD69, increased levels of NKp46, decreased levels of CD25, decreased levels of CD57, decreased levels in CD56, decreased levels of Ki67 and decreased frequency of effector cells with respect to the reference values biomarker is indicative that the subject suffers or is predisposed to CFS.

In a preferred embodiment, the frequency of Tregs in the CD4+ cell population can be determined by measuring the levels of CD25bright/FoxP3+ cells in said CD4+ T cell population.

In a preferred embodiment, the frequency of effector cells in the CD8+ cell population can be determined by measuring the levels of CD45RA⁻/⁺ CCR7⁻D28⁻CD27⁻ cells in the CD8+ cell population.

In a second step, the method of the invention comprises comparing the levels obtained in a) with the reference values for each of the biomarkers.

In a third step, the method of the invention allows correlating increased or decreased levels of the biomarkers with the presence of CFS or a predisposition thereto. In one embodiment, increased levels of CD69 and NKp46 and decreased levels of CD25 and CD57 with respect to the reference value for each biomarker correlate with the presence of CFS or a predisposition thereto. In another embodiment, increased levels of CD69 and NKp46 in CD56+CD16+ NK cells, decreased levels of CD25 and CD57 in CD56+CD16+ NK cells and increased frequency of Tregs in the CD4+ cell population or CD5 in CD8+ T cells or decreased levels in CD56, Ki67 in CD3+ T cells or in the frequency of effector cells with respect to the reference values for each biomarker is indicative that the subject suffers or is predisposed to suffering CFS.

According to the present invention, the level of a biomarker is considered "increased" when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

Likewise, in the context of the present invention, the level of a biomarker is considered "decreased" when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

### 3. Diagnostic kits

The invention also refers to a kit comprising reagents for assaying the levels of CD25, CD69, NKp46 and CD57 in CD56+CD16+ NK cells. In a preferred embodiment, the kit according to the invention further comprises reagents for assaying the levels of at least one biomarker selected from the group consisting of the level of CD56 in CD3+ T cells, the frequency of Tregs in CD4+ T cells, the level of Ki67 in CD4+ T cells, the level of CD5 in CD8+ T cells and the frequency of effector cells in CD8+ T cells.

In another embodiment, the kit of the invention comprises reagents for determining the levels of CD25, CD69, NKp46 and CD57 in CD56+CD16+ NK cells and the levels of any combination of any two, any three or any four markers selected from the group consisting of the level of CD56 in CD3+ T cells, the frequency of Tregs in CD4+ T cells, the level of Ki67 in CD4+ T cells, the level of CD5 in CD8+ T cells and the frequency of effector cells in CD8+ T cells. Suitable kits include reagents for determining the levels of any combination of markers as defined in detail in the context of the methods for diagnosing or for determining predisposition to CFS.

In a preferred embodiment, the kit of the invention comprises reagents for determining the levels of CD25, CD69, NKp46 and CD57 in CD56+CD16+ NK cells, the levels of CD56 in CD3+ T cells, the frequency of Tregs in CD4+ cells, the levels of Ki67 in CD4+ cells and the frequency of effector cells in CD8+ T cells. In yet another embodiment, the kit of the invention comprises reagents for determining levels of CD25, CD69, NKp46 and CD57 in CD56+CD16+ NK cells, the levels of CD56 in CD3+ T cells, the levels of Ki67 in CD4+ cells and the frequency of effector cells in CD8+ T cells.

Additionally, the kits of the invention can contain instructions for the simultaneous, sequential or separate use of its different components. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instructions.

Thus, the reagents of the kit include compounds that bind specifically to the biomarker proteins. Preferably, said compounds are antibodies, aptamers or fragments thereof. More preferably, said compounds are antibodies or fragments thereof.

The antibodies of the kit of the invention can be used according to techniques known in art for determining protein expression levels such as, for example, flow cytometry, Western blot, ELISA, RIA, competitive EIA, DAS-ELISA, techniques based on the use of biochips, protein microarrays, or assays of colloidal precipitation in reactive strips.

The antibodies can be fixed to a solid support such as a membrane, a plastic or a glass, optionally treated to facilitate the fixation of said antibodies to the support. Said solid support may comprise, at least, a set of antibodies which specifically recognize the first biomarker panel and at least one biomarker of the second biomarker panel, and which can be used for detecting the levels of expression of said biomarker.

Additionally, the kits of the invention may comprise reagents for detecting proteins encoded by constitutive genes. The availability of said additional reagents allows normalizing the measurements performed in different samples (for example, the sample to be analyzed and the control sample) to rule out that the differences in the expression of the biomarkers are due to a different quantity of total protein amount in the sample more than the real differences in the relative levels of expression. The constitutive genes in the present invention are genes that are always active or being transcribed constantly and which encode for proteins that are expressed constitutively and that carry out essential cellular functions. Proteins that are expressed constitutively and can be used in the present invention include, without limitation, β-2-microglobulin (B2M), ubiquitin , 18-S ribosomal protein, cyclophilin, GAPDH, PSMB4, tubulin, and actin.

In a preferred embodiment, the reagents for assaying the levels of the different biomarkers comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for assaying the levels of CD25, CD69, NKp46 and CD57 in CD56+CD16+ NK cells, the reagents specific for said biomarkers (i.e. antibodies which bind specifically to CD25, CD69, NKp46 and CD57) comprise at least 10%, at least 20%, at least 30%, at least 40%, at least 50%, at least 60%, at least 70%, at least 80%, at least 90%, or at least 100% of the antibodies present in the kit.

In another embodiment, the invention refers to the use of a kit of the invention for the diagnosis of CFS or for the predisposition to CFS.

### 4. Methods for the treatment and prevention of CFS

In another embodiment, the invention relates to a reagent for inhibiting the expression or activity of at least one biomarker selected from the group consisting of CD69 in CD56+CD16+ NK cells, NKp46 CD56+CD16+ NK cells and CD5 in CD8+ T cells for use in the treatment or prevention of CFS in a subject in need thereof. In a particular embodiment, the reagent for use according to the invention further comprises a reagent for inhibiting the frequency of Tregs in CD4+ cells.

In another embodiment, the invention relates to a method for the treatment or prevention of CFS in a subject in need thereof which comprises the administration to said subject of a reagent for inhibiting the expression or activity of at least one biomarker selected from the group consisting of CD69 in CD56+CD16+ NK cells, NKp46 in CD56+CD16+ NK cells and CD5 in CD8+ T cells. In another aspect, the method for the treatment of the invention further comprises the administration to the subject of a reagent for inhibiting the frequency of Tregs in CD4+ cells.

In a further embodiment, the invention relates to a reagent for inducing the expression or activity of at least one biomarker selected from the group consisting of CD25 in CD56+CD16+ NK cells, CD57 CD56+CD16+ NK cells, CD56 in CD3+ T cells and Ki67 in CD4+ T cells for use in the treatment or prevention of CFS in a subject in need thereof. In a particular embodiment, the reagent for use according to the invention further comprises a reagent for increasing the frequency of effector cell in CD8+ T cells.

In a further embodiment, the invention relates to a method for the treatment or prevention of CFS in a subject in need thereof which comprises the administration to said subject of a reagent for inducing the expression or activity of at least one biomarker selected from the group consisting of CD25 in CD56+CD16+ NK cells, CD57 in CD56+CD16+ NK cells, CD56 in CD3+ T cells and Ki67 in CD4+ T cells. In a particular aspect, the method further comprises the administration to the subject of a reagent for increasing the frequency of effector cell in CD8+ T cells.

In an additional embodiment, the invention relates to a reagent for inhibiting the expression or activity of at least one biomarker selected from the group consisting of CD69, NKp46 and CD5 and ii) a reagent for increasing the expression or activity of at least one biomarker selected from the group consisting of CD25, CD57, CD56 and Ki67 for use in the treatment or prevention of CFS in a subject in need thereof. The use of said reagents can be simultaneous or sequential.

In an additional embodiment, the invention relates to a method for the treatment or prevention of CFS in a subject in need thereof which comprises the administration to said subject of: i) a reagent for inhibiting the expression or activity of at least one biomarker selected from the group consisting of CD69 in CD56+CD16+ NK cells, NKp46 in CD56+CD16+ NK cells and CD5 in CD8+ T cells and ii) a reagent for inducing the expression or activity of at least one biomarker selected from the group consisting of CD25 in CD56+CD16+ NK cells, CD57 in CD56+CD16+ NK cells, CD56 in CD3+ T cells and Ki67 in CD4+ T cells. The administration can be simultaneous or sequential.

According to the present invention, several assays can be used to analyze the activity of NKp46, CD69, CD5, CD25, CD57, CD56 and Ki67 markers. The NKp46 activity can be analyzed, for example, determining the induction of the lysis of infected monocytes *(See* Vankayalapati R.et al, The Journal of Immunology April 1, 2002); the CD5 activity can be determined by analyzing the resistance to apoptosis *(See* Bikah G. et al Science. 1996 Dec 13;274(5294):1906-9); CD69 activity can be determined by assaying the production of TGF-β by activated leukocytes *(See* Sancho D. et al., J Clin Invest. 2003;112(6):872―882*)*: the activity of CD25 can be determined by assaying the binding of IL-2 and IL-10 production from CD4 cells *(See* Amy A. Caudy et al., The Journal of Allergy and Clinical Immunology Vol.119, Issue 2 , Pag. 482-487, 2007); the activity of CD57 can be determined by analyzing CD8+ T cells resistance to spontaneous and CD95/Fas-induced apoptosis *(See* Petrovas C. et al., J Immunol. 2009 July 15; 183(2): 1120―1132.); the activity of CD56 can be analyzed using cell-cell adhesion assays *(See* Rutishauer V, et 1., Science 1 April 1988: Vol. 240 no. 4848 pp. 53-57) and finally the activity of Ki67 can be assayed measuring the cell proliferation (*See* Schlüter C. et al., J Cell Biol. 1993 Nov;123(3):513-22).

Reagents suitable for inhibiting the frequency of Tregs in CD4+ cells include compounds suitable for inhibiting CD25. Inhibitory agents suitable for use according to the present invention (e.g. CD69, NKp46, CD5 or CD25 inhibition) include, but are not limited to, antisense oligonucleotides, interference RNAs (siRNAs), catalytic RNAs, specific ribozymes, and inhibitory antibodies or nanobodies.

### A. Antisense oligonucleotides

An additional aspect of the present invention relates to the use of isolated "antisense" nucleic acids to inhibit the expression (i.e. inhibiting the DNA transcription or mRNA translation) of a nucleic acid which encodes the CD69, NKp46, CD5 or CD25 biomarkers. The antisense nucleic acids can be bound to the potential target by means of conventional base complementarity or, for example, in the case of binding to double stranded DNA, through specific interaction in the large groove of the double helix. Generally, these methods refer to a range of techniques generally used in the art and include any method which is based on the specific binding to oligonucleotide sequences.

An antisense construct of the present invention can be distributed, for example, as an expression plasmid which when transcribed in a cell produces RNA complementary to at least one unique part of the cellular mRNA encoding CD69, NKp46, CD5 or CD25. Alternatively, the antisense construct is an oligonucleotide probe generated *ex vivo* which when introduced into the cell inhibits the expression of the gene or the translation of the mRNA of the gene with which it hybridizes. Preferably, the oligonucleotide probes are modified oligonucleotides resistant to endogenous nucleases (e.g. exonucleases, endonucleases) stable *in vivo.* Examples of nucleic acids molecules for use herein as antisense oligonucleotides, include, but are not limited to, DNA analogs of phosphoramidate, phosphothionate and methylphosphonate. *See* Hogan M, et al., US 5,176,996, Matteucci M, US 5,264,564, and Froehler B, US 5,256,775. The general approximations for constructing oligomers useful in the antisense therapy have been described in the art. *See* Van der Krol P, et al., BioTechniques 1988; 6:958-976 and Stein C, et al., Cancer Res. 1988; 48:2659-2668. Preferably, the oligodeoxyribonucleotide regions utilized are located between -10 and +10 of the translation starting site of the target gene. The antisense approximations involve the oligonucleotide design (i.e. DNA or RNA) that are complementary to the mRNA encoding the target polypeptide. The antisense oligonucleotide will bind the transcribed mRNA thus preventing its translation into the target polypeptide.

The oligonucleotides which are complementary to the 5' end of the mRNA such as, for example, the non-translated 5' sequence up to and including the start codon AUG of the target gene, must function in the most efficient manner to inhibit translation. However, the sequences complementary to the non-translated 3' end of the mRNA are also efficient for inhibiting mRNA translation. *See* Wagner R, et al., Nature 1994; 372: 333-335. Therefore, complementary oligonucleotides could be used at the non-coding 5' or 3' regions of the mRNA of the target gene to inhibit its translation. Preferably, the oligonucleotides complementary to the non-translated 5' region of the mRNA include the complement to the start codon AUG. The oligonucleotides complementary to the coding region of the mRNA are generally less efficient translation inhibitors, but could also be used according to the present invention. Preferably, the antisense nucleic acids must be at least six nucleotides long.

*In vitro* studies are performed customarily to quantify the capacity of the antisense oligonucleotides for inhibiting gene expression. Preferably, these studies use controls which distinguish between antisense gene inhibition and the non-specific biological effects attributable to the oligonucleotides. More preferably, the studies compare the levels of target RNA or protein with an internal RNA or protein control reference. Preferably, the control oligonucleotide is approximately of the same length as the target oligonucleotide and does not differ significantly from the antisense sequence, so that it does not hybridize with the target sequence.

The antisense oligonucleotide can be a single or double stranded DNA or RNA or chimeric mixtures or derivatives or modified versions thereof. The oligonucleotide can be modified in the phosphate backbone, base or sugar groups in order to, for example, improve its stability or enhance its hybridization capacity. The oligonucleotide may include other bound groups, such as peptides (e.g. for directing them to the surface receptors in the host cells) or agents for facilitating transport through the cell membrane or the blood-brain barrier, and intercalating agents. *See* Letsinger R, et al., Proc. Natl. Acad. Sci. USA 1989; 86:6553-6556, Lemaitre M, et al., Proc. Natl. Acad. Sci. USA 1987; 84:648-652, Pardridge W, et al., WO 1989/010134, Tulli R, et al., WO 1988/009810, and Zon G, et al., Pharm. Res. 1988; 5:539-549. The oligonucleotide can be conjugated to another molecule such as, for example, a peptide, a transporting agent, or hybridization triggered cleaving agent.

The antisense oligonucleotides may comprise at least one modified base group. The antisense oligonucleotide may also comprise at least one a modified sugar group such as, for example, arabinose, 2-fluoroarabinose, xylulose, and hexose. The antisense oligonucleotide may also contain a backbone similar to a neutral peptide. Such molecules are known as peptide nucleic acid (PNA) oligomers and have been described previously. *See* Perry-O'Keefe H, et al., Proc. Natl. Acad. Sci. USA 1996; 93:14670-14675.

In yet another embodiment, the antisense oligonucleotide comprises at least one modified phosphate backbone. In yet another embodiment, the antisense oligonucleotide is an α-anomeric oligonucleotide.

While antisense oligonucleotides complementary to the coding region of the target mRNA sequence can be used, those complementary to the transcribed non-translated region can also be used.

In some cases, it may be difficult to reach the sufficient intracellular concentrations of the antisense oligonucleotide to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (i.e. promoter, enhancer) to form triple helix structures preventing gene transcription in the target cells. *See* Hélène C, et al., Anticancer Drug Des. 1991; 6(6):569-584.

### B. siRNA

Small interfering RNA or siRNA are agents which are capable of inhibiting the expression of a target gene by means of RNA interference. A siRNA can be synthesized chemically or *in vivo* in the target cell, or by means of *in vitro* transcription. Typically, the siRNA consists of a double stranded RNA between 15 and 40 nucleotide long. In addition, it may contain a 3' or 5' protruding region of 1 to 6 nucleotides. The length of the protruding region is independent of the total length of the siRNA molecule. The siRNA acts by means of degrading or silencing the target mRNA after transcription.

The siRNA of the invention are substantially homologous to the mRNA of the CD69, NKp46, CD5 or CD25 encoding genes or to the gene sequences which encode said proteins. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as:
1) siRNA in which the bonds between the nucleotides are different than those that appear in nature (e.g. phosphorothionate bonds),
2) conjugates of the RNA strand with a functional reagent (e.g. a fluorophore),
3) modifications to the ends of the RNA strands, particularly of the 3' end by means of the modification with different hydroxyl functional groups in 2' position,
4) nucleotides with modified sugars (e.g. O-alkylated residues on 2' position such as 2'-O-methylribose or 2'-O-fluororibose), and
5) nucleotides with modified bases (e.g. halogenated bases such as 5-bromouracil and 5-iodouracil or alkylated bases such as 7-methylguanosine).

The siRNA can be used as is (i.e. in the form of a double stranded RNA with the aforementioned characteristics). Alternatively, the use of vectors containing the sense and antisense strand sequence of the siRNA is possible under the control of suitable promoters for the expression thereof in the cell of interest.

Vectors suitable for expressing siRNA include those where the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

Alternatively, the use of vectors in which each of the strands forming the siRNA is derived from the transcription of a different transcriptional unit is possible. These vectors are divided into divergent and convergent transcription vectors. In divergent transcription vectors, the transcriptional units encoding each of the DNA strands forming the siRNA are located in tandem such that the transcription of each DNA strand depends on its own promoter, which may be the same or different. *See* Wang J, et al., Proc. Natl. Acad. Sci. USA 2003; 100:5103-5106 and Lee N, et al., Nat. Biotechnol. 2002; 20:500-505. In convergent transcription vectors, the DNA regions giving rise to the siRNA form the sense and antisense strands of a DNA region which are flanked by two reverse promoters. After the transcription of the sense and antisense RNA strands, the latter will form the hybrid for forming a functional siRNA. Convergent transcription vectors include, but are not limited to, those based on the use of 2 U6 promoters, a mouse U6 promoter and a human H1 promoter, and a human U6 promoter and a mouse H1 promoter. *See* Tran N, et al., BMC Biotechnol. 2003; 3:21, Zheng L, et al., Proc. Natl. Acad. Sci. USA 2004; 101:135-140, Kaykas A, et al., BMC Cell Biol. 2004; 5:16, and Framer A, WO 2005/026322.

Promoters suitable for use in the expression of siRNA by convergent or divergent expression vectors include any promoter or pair of promoters compatible with the cells in which the siRNA is to be expressed. Thus, promoters suitable for the present invention include, but are not limited to, constitutive promoters, such as those derived from the genomes of eukaryotic viruses (e.g. polyoma virus, adenovirus, SV40, CMV, avian sarcoma virus, hepatitis B virus), the metallothionein gene promoter, the thymidine kinase gene promoter of the herpes simplex virus, retrovirus LTR regions, the immunoglobulin gene promoter, the actin gene promoter, the EF-1 alpha gene promoter as well as inducible promoters in which protein expression depends on the addition of a molecule or an exogenous signal such as the tetracycline system, the NFkappaB/UV light system, the Cre/Lox system and the heat shock gene promoter, regulable RNA polymerase II promoters as well as specific tissue promoters (e.g. PSA promoter). In a preferred embodiment, the promoters are RNA polymerase III promoters which act constitutively. *See* Lewin A, et al., WO 2006/135436 and Chiu R, et al., WO 2006/012221. The RNA polymerase III promoters are found in a limited number of genes such as 5S RNA, tRNA, 7SL RNA and U6 snRNA. Unlike other RNA polymerase III promoters, type III promoters do not require any intragenic sequence but rather requires sequences in 5' direction comprising a TATA box in positions -34 and - 24, a proximal sequence element or PSE between -66 and -47 and, in some cases, a distal sequence element or DSE between positions -265 and -149. In a preferred embodiment, the type III RNA polymerase III promoters are the human or murine H1 and U6 gene promoters. In a yet more preferred embodiment, the promoters are 2 human or murine U6 promoters, a mouse U6 promoter and a human H1 promoter or a human U6 promoter and a mouse H1 promoter.

The siRNA can be generated intracellularly from shRNA (short hairpin RNA). shRNA is a sequence of RNA that makes a tight hairpin turn that can be used to silence target gene expression via RNA interference. The shRNAs can be encoded by plasmids or viruses, particularly retroviruses, under the control of a promoter. Promoters suitable for expressing shRNA are those indicated herein for expressing siRNA.

Vectors suitable for expressing siRNA and shRNA include prokaryotic expression vectors (e.g. pUC 18, pUC 19, Bluescript and the derivatives thereof, mp18, mp19, pBR322, pMB9, CoIE1, pCR1, RP4), phages, shuttle vectors (e.g. pSA3 and pAT28), yeast expression vectors (e.g. 2-micron plasmid type vectors, integration plasmids, YEP vectors, centromeric plasmids), insect cell expression vectors (e.g. pAC series vectors, pVL series vectors), plant expression vectors (e.g. pIBI, pEarleyGate, pAVA, pCAMBIA, pGSA, pGWB, pMDC, pMY, pORE series vectors) and viral vectors (e.g. adenovirus, adeno-associated viruses, retroviruses, lentiviruses) higher eukaryotic cell expression vectors or non-viral vectors (e.g. pcDNA3, pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL, pKSV-10), pBPV-1, pML2d and pTDT1. In a preferred embodiment, the vectors are lentiviral vectors.

The siRNA and shRNA of the invention can be obtained using a series of techniques known in the art. The region of the nucleotide sequence taken as basis for designing the siRNA is not limiting and may contain a region of the coding sequence or may contain alternatively sequences of the non-translated 5' or 3' regions. Preferably, the sequences are between 25 and 50 nucleotides long and are located downstream the start codon. One way of designing a siRNA involves the identification of the AA(N19)TT motifs wherein N can be any nucleotide in the CD69, NKp46 or CD5 gene sequence, and the selection of those having a high G/C content. If said motif is not found, it is possible to identify the NA(N21) motif wherein N can be any nucleotide.

### C. DNA Enzymes

In another aspect, the invention also contemplates the use of DNA enzymes to inhibit the expression of the CD69, NKp46, CD5 or CD25 coding gene of the invention. DNA enzymes incorporate some of the mechanistic features of both antisense and ribozyme technologies. DNA enzymes are designed to recognize a particular target nucleic acid sequence in a manner similar to antisense oligonucleotide. However, instead of interfering with the expression of the target nucleotide sequence, the enzyme catalyzes and cleaves it.

### D. Ribozymes

In another aspect, the invention includes ribozyme molecules designed for cleaving catalytically mRNA encoding CD69, NKp46, CD5 or CD25, thus inhibiting their expression. Ribozymes are enzymatic RNA molecules capable of catalyzing specific RNA sequences. *See* Rossi J, Curr. Biol. 1994; 4:469-471. The mechanism of ribozyme action involves a specific hybridization of a ribozyme molecule sequence to a complementary target RNA followed by an endonucleolytic cleavage event. The composition of the ribozyme molecules includes, preferably, one or more sequences complementary to the target mRNA, and a sequence known for cleaving the mRNA or a functionally equivalent thereof. *See* Cech T, et al., US 5,093,246. The ribozymes used in the present invention include hammer-head ribozymes, and endoribonuclease RNA ("Cech type ribozymes"). *See* Zaug A, et al., Science 1984; 224:574-578.

Alternatively, the ribozymes can be formed by modified oligonucleotides (e.g. to improve stability, targeting). They can be distributed to cells expressing the target gene *in vivo.* A preferred distribution method involves using a DNA construct which "encodes" the ribozyme under the control of a strong constitutive pol III or pol II promoter so that the transfected cells will produce sufficient amounts of it, destroy the endogenous target messengers and inhibit their translation. Since ribozymes are catalytic, a low intracellular concentration is preferred for its efficiency.

### E. Inhibitory antibodies

In the context of the present invention, "inhibitory antibody" is understood as any antibody capable of binding specifically to the CD69, NKp46, CD5 or CD25 protein and inhibiting one or more of the functions of said protein. The antibodies can be prepared by using several methods known in the art. Thus, monoclonal and polyclonal antibodies may be prepared and employed according to the present invention. *See* Köhler G, et al., Nature 1975; 256:495-497. Suitable antibodies include intact antibodies comprising a variable antigen binding region and a constant region, "Fab", "F(ab')2" and "Fab'", Fv, scFv fragments, diabodies and bispecific antibodies. Once antibodies with CD69, NKp46, CD25 or CD5 protein binding capacity are identified, those capable of inhibiting the activity of said proteins will be selected using an inhibitory agent identification assay. For instance, anti NKp46 antibodies can be used to deplete NKp46+ NK cells. *See* Gürcan H, et al., Int. Immunopharmacol. 2009; 9(1):10-25. Zolimomab aritox is an anti-CD5 antibody which can be used to inhibit CD5. Reactants aimed to reduce the numbers or the function of Treg cells, such as anti CD25 antibody basiliximab or anti TGF-beta antibodies have also been reported. *See* Zhao T, et al., Transplant Proc. 2012; 44(1):175-178 and Trachtman H, et al., Kidney Int. 2011; 79(11):1236-1243.

### F. Inhibitory peptides

In another aspects, inhibitory peptides capable of binding to the CD69, NKp46, CD25, or CD5 protein and inhibiting its activity can be used according to the invention.

### G. Other inhibitory compounds

Other CD69, NKp46 or CD5 inhibitory compounds suitable for use in the present invention include without limitation:
1) Cyclosporin A and La3+ as CD69 inhibitors. *See* Ortiz A, et al., J. Rheumatol. 2000; (10):2329-2338, Aussel C, Biochem J. 1996; 313(Pt3):909-913, and Brefeldin A, et al., J. Immunol. Methods 1999; 224(1-2):69-76).
2) L-kynurenine, histone deacetylase inhibitors (HDACi), phenylbutyrate, valproic acid, derivatives of hydroxamic acid (e.g. PXD101, suberoylanilide hydroxamic acid (SAHA), trichostatin A), the benzamide derivative MS-275, and the naturally occurring depsipeptide FR901228 as NKp46 inhibitors. *See* Della Chiesa M, Blood 2006; 108(13):4118-4125 and Ogbomo H, et al., FEBS Lett. 2007; 581(7):1317-1222.
3) IL-4 as CD5 inhibitor. *See* Hidaka T, et al., Clin. Exp. Immunol. 1992; 89(2): 223-229.

### H. Compounds and systems for inducing gene expression or increasing protein activity

Any method of enhancing gene expression known in the art can be used herein. For instance, a recombinant expression vector comprising a polynucleotide encoding CD25, CD57, CD56 or Ki67 or functional equivalent thereof operably linked to a promoter suitable for expression in a target cell can be prepared. Alternatively, a regulating sequence or functional equivalent thereof can be vectorized to enhance the expression of the CD25, CD57, CD56 or Ki67 endogenous genes. There are several techniques disclosed in the art for introducing nucleic acids into viable cells. Examples of said methods include, without limitation, chemical-based methods (e.g. highly branched organic compounds, dendrimers, liposomes, cationic polymers such as DEAE-dextran or polyethylenimine), non-chemical methods (e.g. electroporation, optical transfection), particle-based methods (e.g. gene gun, magnetofection or magnet-assisted transfection), or viral methods (e.g. adenovirus, retrovirus or lentivitrus).

The promoters that can be used for regulating the transcription of the polynucleotides of the invention can be constitutive promoters (i.e. promoters directing the transcription at a basal level) or inducible promoters (i.e. where the transcriptional activity requires an external signal). Constitutive promoters suitable for regulating transcription include, but are not limited to, the CMV promoter, the SV40 promoter, the DHFR promoter, the mouse mammary tumor virus (MMTV) promoter, the 1a elongation factor (EF1a) promoter, the albumin promoter, the ApoA1 promoter, the keratin promoter, the CD3 promoter, the immunoglobulin heavy or light chain promoter, the neurofilament promoter, the neuron specific enolase promoter, the L7 promoter, the CD2 promoter, the myosin light chain kinase promoter, the HOX gene promoter, the thymidine kinase promoter, the RNA polymerase II promoter, the MyoD gene promoter, the phosphoglyceratekinase (PGK) gene promoter, the low density lipoprotein (LDL) promoter, and the actin gene promoter. The inducible promoters that can be used in the context of the present invention are those responding to an inducer agent showing zero or negligible basal expression in the absence of an inducer agent and which are capable of promoting the activation of a gene located in the 3' position. Depending on the type of inducer agent, the inducible promoters are classified as Tet on/off promoters, Pip on/off promoters, antiprogestin-dependent promoters, ecdysone-dependent promoters, a metallothionein-dependent promoter, or rapamycin-dependent promoters. *See* Gossen M, et al., Proc. Natl. Acad. Sci. USA 1992; 89:5547-5551, Gossen M, et al., Science 1995; 268:1766-1769, Rossi F, et al., Curr. Opin. Biotechnol. 1998; 9:451-456, Fussenegger M, et al., US 6,287,813, Horwitz K, et al., US 20040132086, Christopherson K, et al., Proc. Natl. Acad. Sci. USA 1992; 89:6314-6318, No D, et al., Proc. Natl. Acad. Sci. USA 1996; 93:3346-3351, Suhr S, et al., Proc. Natl. Acad. Sci. USA 1998; 95:7999-8004, Evans R, et al., WO 1997/038117, Kushner P, et al., WO 1986/004920, and Rivera V, et al., Nat. Med. 1996; 2:1028-1032.

Vectors suitable for expressing the polynucleotide encoding CD25, CD57, CD56 or Ki67 include vectors derived from prokaryotic expression vectors (e.g. pUC18, pUC19, Bluescript and derivatives thereof, mp18, mp19, pBR322, pMB9, ColEl, pCR1, RP4), phages and shuttle vectors (e.g. pSA3, pAT28), yeast expression vectors (e.g. 2-micron type plasmid vectors, integration plasmids, YEP vectors, centromeric plasmids), insect cell expression vectors (e.g. pAC series vectors, pVL series vectors), plant expression vectors (e.g. pIBI, pEarleyGate, pAVA, pCAMBIA, pGSA, pGWB, pMDC, pMY, pORE series vectors), viral expression vectors (e.g. adenoviruses, adeno-associated, retroviruses, lentiviruses), higher eukaryotic cell expression vectors or non-viral vectors (e.g. pSilencer 4.1-CMV (Ambion®, Life Technologies Corp., Carlsbad, CA, US), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL, pKSV-10), pBPV-1, pML2d, and pTDT1.

CD25 and Ki-67 inducers compounds suitable for use in the present invention include, but are not limited to:
1) IL-4, Epstein-Barr virus (EBV) nuclear antigen 2 (EBNA-2) gene, IL-12, and GM-CSF and IL-3 or IL-5 as CD25 inducers. *See* McKay C, et al., Cytokine 1996; 8:305-312, Harada S, et al., Microbiol Immunol. 1992; 36:479-494, Nguyen T, et al., Eur J Immunol. 2000; 30:1445-1452, and Chihara J, et al., Nihon Rinsho 1993; 51:581-587.
2) Vigabatrin as Ki67 inducer compound. *See* Mesa F, et al., J. Cell Biochem. 2005; 94:744-762.

### 5. Therapeutic kits

In another embodiment, the invention also refers to a kit comprising the reagents for inducing or inhibiting the expression or activity of the biomarkers of the invention.

In a preferred embodiment, the reagents for inducing or inhibiting the expression or activity of the biomarkers of the invention comprise at least 10%, at least 20%, at least 30%, at least 40%, at least 50%, at least 60%, at least 70%, at least 80%, at least 90%, or at least 100% of the total amount of reagents forming the kit.

The kit may contain additional components for maintaining the reagents properly stored. Suitable materials for preparing such components include, but are not limited to, glass, plastic (e.g. polyethylene, polypropylene, polycarbonate), bottles, vials, paper, and sachets. Additionally, the kits of the invention can contain instructions for the simultaneous, sequential or separate use of its different components. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instructions.

In another embodiment, the invention relates to a composition or kit-of-parts comprising, together or separately, a reagent for inhibiting the expression or activity of at least one biomarker selected from the group consisting of CD69, NKp46 and CD5 for use in the treatment or prevention of CFS in a subject in need thereof and a reagent for inducing the expression or activity of at least one biomarker selected from the group consisting of CD25, CD57, CD56 and Ki67 for use in the treatment or prevention of CFS in a subject in need thereof. The composition or kit-of parts according to the present invention allows for the separate or combined administration of the two types of reagents. For example, the administration of the reagent or reagents for inhibiting the expression or activity of at least one biomarker selected from the group consisting of CD69, NKp46 and CD5 may be performed first, followed by the administration of a reagent or reagents for inducing the expression or activity of at least one biomarker selected from the group consisting of CD25, CD57, CD56 and Ki67. Alternatively, the administration of a reagent or reagents for inducing the expression or activity of at least one biomarker selected from the group consisting of CD25, CD57, CD56 and Ki67 reagent may be performed first, followed by the administration of a reagent or reagents for inhibiting the expression or activity of at least one biomarker selected from the group consisting of CD69, NKp46 and CD5. Alternatively, the administration of the reagent or reagents for inhibiting the expression or activity of at least one biomarker selected from the group consisting of CD69, NKp46 and CD5 may be performed at the same time as the administration of a reagent or reagents for inducing the expression or activity of at least one biomarker selected from the group consisting of CD25, CD57, CD56 and Ki67.

The compositions or kit-of-parts of the invention may be administered using any suitable route including, but not limited to, the intravascular, intratumoral, intracranial, intraperitoneal, intrasplenic, intramuscular, subretinal, subcutaneous, mucosal, topical or oral route.

In another embodiment, the invention relates to a kit for use in the treatment or prevention of CFS in a subject in need thereof.

This invention is further illustrated by the following examples which should not be construed as limiting. The contents of all references, patents and published patent applications cited throughout this application are incorporated herein in their entirety by reference.

### General Procedures

### 1. Subject characteristics

A limited number of CFS patients (N=22) and healthy donors (N=30) were selected according to the Fukuda criteria. *See* Fukuda, 1994, *supra.* The main characteristics of the individuals recruited for the assay are summarized in Table 1. Both control and CFS groups showed similar median age values (38 and 44 years, respectively, p=ns, Mann-Whitney test) and were mostly composed by females (55% and 73% respectively, p=ns, Fisher exact test). Patients suffering from CFS showed a median time from certified diagnosis of 3 years. Additional factors that may modulate immune function such as comorbidities and polypharmacy associated with CFS were also evaluated. Main drugs and clinical complications are also described in Table 1. Three individuals in the CFS group were excluded from the analysis, one due to a B cell lymphocytosis (B cells represented 24% of lymphocytes showing a total B cell count of 435 cells/µL, with more than 85% of cells showing a IgD⁺IgM⁻CD23⁺CD27⁺CD5⁺CD38⁻ phenotype), one due to an IgA deficiency and a third due to sample unavailability.

**Table 1**

| Main characteristics of the subjects analyzed | | | | | |
|---|---|---|---|---|---|
| | | | SFC (N=22) | HD (N=30) | *P-value* (Mann-Whitney) |
| | | | | | |
| Age (years, Median, IQR) | | | 44 [40-50] | 38 [33-52] | *ns* |
| Gender (% of female) | | | 73 | 55 | *ns* |
| Time from diagnosis (Years, Median, IQR) | | | 3 [3-5] | - | |
| Reported onset with viral infection (%) | | | 53 | - | |
| Pharmacy (%) | | | | - | |
| | Analgesics | | 42 | | |
| | Anxiolytics | | 21 | | |
| | Antidepressants | | 16 | | |
| | Homeopathy | | 58 | | |

| Comorbidities (%) | | | | - | |
|---|---|---|---|---|---|
| | Anxiety | | 74 | | |
| | Fibromyalgia | | 47 | | |
| | Mult. Chem. Sensitivity | | 32 | | |
| | Autoimmunity | | 10 | | |
| | Tendinopathy | | 37 | | |

### 2. Sample collection

Blood was collected by venipuncture in EDTA vacutainer tubes (BD Biosciences Corp., Franklin Lakes, NJ, US). A single blood sample was drawn from all participants. A 5 mL aliquot was reserved for immediate immunophenotype; the remaining blood was processed for plasma and PBMC preparation as described. *See* Barretina J, et al., AIDS 2004; 18:1673-1682. Plasma was obtained by centrifugation of blood at 1200x g for 10 minutes and stored at ―80 °C. Peripheral blood mononuclear cells (PBMC) were obtained from cell concentrates layered on Ficoll-Hypaque density gradients (Atom Reactiva S.A., Barcelona, ES) and centrifuged for 30 minutes at 800xg. Cellular interface was collected, washed twice in PBS and resuspended in RPMI culture medium for proliferation, NK cell activity and cell death assays, or cryopreserved in DMSO/FCS ratio 1:10.

### 3. Determination of absolute counts of B, T and NK cells

Absolute counts of B, T and NK cells were analyzed in two steps by flow cytometry. Firstly, the absolute lymphocyte count was determined using an anti-CD45― V450 antibody (BD Biosciences Corp., Franklin Lakes, NJ, US) in combination with the perfect-count microspheres reagent (Cytognos S.L., Salamanca, ES), following the manufacturer instructions. Secondly, the percentage of the different lymphocyte subsets was determined using the following antibody combination: CD45―V450, CD19― AmCyan, CD3―APC-Cy7, CD4―APC, CD8―PerCP, CD56―PE and CD16―FITC (BD Biosciences Corp., Franklin Lakes, NJ, US). Absolute count of each cellular population was calculated as follows: (X*Y)/100, where X is the percentage of each subset and Y is the absolute count of lymphocytes.

### 4. Immunophenotyping

Freshly obtained blood was incubated for 15 minutes at room temperature with different antibody combinations in order to characterize B, T and NK cells populations. *See* Table 2. All antibodies were from BD Biosciences unless indicated. Cells were then treated for 15 minutes in FACS Lysing solution (BD Biosciences Corp., Franklin Lakes, NJ, US), washed in PBS and fixed in PBS containing 1% formaldehyde (Sigma-Aldrich Co., Saint Louis, MO, US), before data acquisition in an LSRII flow cytometer.

**Table 2**

| | | | | | | | | | | |
|---|---|---|---|---|---|---|---|---|---|---|
| Antibody combinations used for the phenotypic characterization of T, NK, B cells and for the determination of absolute counts | | | | | | | | | | |

| Antibody combinations/ | | | | | | | Fluorochrome | | | |
|---|---|---|---|---|---|---|---|---|---|---|
| CELL TYPE | Phenotype | Tube # | V450 / Pacific blue | V500/ Am Cyan | FITC | PE | APC / Alexa Fluor 647 | PerCP/ PerCP-Cy5 | PE-Cy7 | APC-cy7 |
| T cells | Thymic output/ exhaustion | T1 | | | CD95 | PD-1 | CD4 | HLA-DR | CD8 | CD3 |
| | | T2 | | | CD45RA | CD31 | CD4 | CD38^{d} | CD8 | CD3 |
| | | T3 | | | | | CD4 | | CD8 | CD3 |
| | CD8 activation | T4 | CD4 | | HLA-DR | | CD45RO | CD38^{d} | CD8 | CD3 |
| | | T5 | CD4 | | | | | | CD8 | CD3 |
| | Main subsets/ senescence | T6 | CD45RA | CD8 | CD57^{e} | CD28 | CD27 | CD4 | CCR7 | CD3 |
| | | T7 | | CD8 | | | | CD4 | | CD3 |
| | Treg/ Prolif./ Anergy | T8 | CD4 | CD8 | Ki67 | FoxP3 ^{e} | CD127^{c} | CD5^{d} | CD25 | CD3 |
| | | T9 | CD4 | CD8 | IRR IgG | IRR IGG2a ^{e} | | | | CD3 |
| NK cells | Activation receptor profile | NK1 | CD69 | CD3^{b}/ CD19^{b} | CD107a | Nkp44 | CD57^{e} | CD25 | CD56 | CD16 |
| | | NK2 | NKp4 6^{a,ed} | CD3^{b}/ CD19^{b} | CD107a | NKG2 D^{e} | Nkp30^{c} | NKG2A ^{e} | CD56 | CD16 |
| | | NK3 | | CD3^{b}/ CD19^{b} | | | | | CD56 | CD16 |
| B cells | Mature/ Memory/ Transitional | B1 | | | CD1c | IgD^{e} | IgM | CD38 | CD27^{e} | CD19 |
| | | B2 | | | CD23 | IgD^{e} | CD5 | CD38 | CD10^{e} | CD19 |
| | | B3 | | | | IgD^{e} | | | | CD19 |
| | Switched memory | B4 | | | CD1c^{e} | IgG^{e} | IgA | CD38 | CD27^{e} | CD19 |
| | | B5 | | | | IgG^{e} | IgA^{e} | | | CD19 |

| | | | | | | | | | | |
|---|---|---|---|---|---|---|---|---|---|---|
| ^{a} Pacific blue-coupled antibody ^{b} AmCyan-coupled antibody ^{c} Alexa Fluor 647-coupled antibody ^{d} PerCP-Cy5.5 coupled antibody ^{e} reagents from sources different than BD Biosciences; NKp46 and CD57 (BioLegend Co., San Diego, CA, US), NKG2A (R&D Systems, Inc., Minneapolis, MN, US), NKG2D and CD1c (Santa Cruz Biotechnology, Inc., Santa Cruz, CA, US), FoxP3, IRR IGG2a CD27, and CD10 (eBioscience, Inc., San Diego, CA, US), IgG and IgA (Jackson ImmunoResearch, Inc., West Grove, PA, US), and CD57 (Beckman Coulter, Inc., Brea, CA, US). | | | | | | | | | | |

An alternative combination to evaluate all relevant T cell parameters in one tube should contain the following:

| CELL TYPE | Phenotype | Tube # | V450 / Pacific blue | V500/ Am Cyan | FITC | PE | APC / Alexa Fluor 647 | PerCP/ PerCP-Cy5 | PE-Cy7 | APC-cy7 |
|---|---|---|---|---|---|---|---|---|---|---|
| T | Treg/Prolif./ Anergy | T8 | CD4 | CD8 | Ki67 | FoxP3^{e} | CD127^{c} | CD5^{d} | CD25 | CD3 |
| T | Control | T9 | CD4 | CD8 | IRR IgG | IRR IGG2a^{e} | | | | CD3 |

The latter approach would allow for the identification of: i) CD4+ cells (CD3+CD4+) and ii) CD8+ cells (CD3+CD8+) and the determination of: iii) CD5- cells (% of CD8+CD3+ cells), iv) Treg cells FoxP3+CD25+ (% of CD4+CD3+ cells), and v) Ki67+ cells (% of CD4+CD3+ cells). Treg cells may also be identified as FoxP3+CD25+ CD127- cells (i.e. % of CD4+CD3+ cells).

An alternative combination to evaluate all relevant NK cell parameters in one tube should contain the following:

| CELL TYPE | Phenotype | Tube # | V450 / Pacific blue | V500/ Am Cyan | FITC | PE | APC / Alexa Fluor 647 | PerCP/ PerCP-Cy5 | PE-Cy7 | APC-cy7 |
|---|---|---|---|---|---|---|---|---|---|---|
| NK | Relevant markers | 1 | CD69 | CD3 | NKp46 | CD19 | CD57 | CD25 | CD56 | CD16 |

The latter approach would allow the removal of B cells (CD19+) for the gating of T cells (CD3+) and NK cells (CD19-/CD3-) and the determination of: i) CD56+ cells (% of CD3+ cells), ii) MFI CD57 in CD56+CD16+ NK cells, iii) CD25+ cells (% of CD56+CD16+ NK cells), iv) CD69+ cells (% of CD56+CD16+ NK cells), and v) NKp46 cells (% of CD56+CD16+ NK cells).

### 5. B cell and T cell proliferation assays

Freshly obtained PBMC were stained with 0.33 µM CFSE (Invitrogen Corp., Carslbad, CA, US) for 5 minutes at room temperature, after extensive washes cells were cultured in RPMI1640 medium supplemented with 10% of FBS (RIO medium) and different activating stimulus for B or T cells. The proliferation capacity of T cells was assayed using 5 µg/mL of PHA (Sigma-Aldrich Co., Saint Louis, MO, US) plus 10 U/mL of IL-2 (F. Hoffmann-La Roche Ltd., Basel, CH). For B cells, 3 µg/mL of endotoxin-free CpG 2006 (InvivoGen Corp., San Diego, CA, US) or 1 µg/mL of R848 (Alexis Biochemicals, Enzo Life Sciences, Inc., Farmingdale, NY, US) were used alone or in combination with 5 µg/mL of F(ab)2 goat anti-human total Igs (Jackson ImmunoResearch Laboratories, Inc., West Grove, PA, US). Four days later, cellular proliferation was assessed by flow cytometry after staining cells with anti CD2―PerCP-Cy5.5, CD5―APC, CD4―V450, CD8―APC-Cy7 and CD19―PE-Cy7 antibodies for T cells or CD3―APC-Cy7, CD19―PE-Cy7, CD14―PerCP (BD Biosciences Corp., Franklin Lakes, NJ, US) and IgD-APC (Miltenyi Biotec GmbH, Bergisch Gladbach, DE) for B cells. Data analysis was performed with the FlowJo software (Tree Star, Inc., Ashland, OR, US), the division and the proliferation indexes were calculated for each condition using best fits provided by the software.

### 6. Cell death assays

Cell death was evaluated by culturing PBMC in of R10 medium at a density of 2x10⁶ PBMC/ml for 24 hours. *See* Massanella, 2010 and Negredo, 2010, *supra.* For T cell death analysis, PBMC were incubated with 40 nM of the potentiometric mitochondrial probe DIOC₆ (Invitrogen Corp., Carslbad, CA, US), 5 µg/mL propidium iodide (Sigma-Aldrich Co., Saint Louis, MO, US), and CD3―APC-Cy7, CD4―APC and CD8―PE-Cy7 antibodies. For B cell death analysis, PBC were incubated with 40 nM of DIOC₆, 0.3 µM of Sytox Blue (Invitrogen Corp., Carslbad, CA, US) and CD19―APC-Cy7, IgD―PE, CD38―PerCP-Cy5.5, CD5―APC (BD Biosciences Corp., Franklin Lakes, NJ, US) and CD27―PE-Cy7 (eBioscience, Inc., San Diego, CA). Cells were acquired in an LSRII flow cytometer; dead cells were identified by their low DIOC₆ staining. *See* Blanco J, et al., J. Leukoc. Biol. 2004; 76: 804-811.

### 7. NK activity assays

Lytic activity of NK cells was assessed in some patients using a flow cytometry based assay previously described with minor modifications. *See* Allegra S, et al., Cytometry A 2006; 69:992-998. To remove monocytes/macrophages from effector cells, 4x10⁶ freshly obtained PBMC were incubated for 1h at 37°C (5% CO₂) in six well plates, harvested suspension cells were considered as the peripheral blood lymphocyte fraction (PBL). eGFP-K562 cells growing exponentially were used as target cells (AIDS Research and Reference Reagent Program, Division of AIDS, NIAID). *See* Kantakamalakul W, et al., J. Immunol. Methods 2003; 272: 189-197. Incubations were performed in duplicate in 96 well plates by seeding a fixed amount (10,000 target cells) alone or with different amounts of PBL ranging from 800,000 to 25,000 in a final volume of 200 µL of complete RPMI medium, covering a range from 80:1 to 2.5:1 effector:target cell ratios. Samples were incubated for 4h at 37°C (5% CO₂) stained for 10 min with 5 µg/mL propidium iodide (PI, Sigma-Aldrich Co., Saint Louis, MO, US) and acquired in a LSRII flow cytometer. For analysis ofNK cell activity, the percentage of dead (PI⁺) eGFP-K652 cells in the gate of eGFP-K562 cells was calculated.

### 8. Soluble CD14

Soluble CD14 (sCD14) levels were quantified in plasma samples using an ELISA assay (Diaclone™, Gen-Probe Inc., San Diego, CA, US). Plasma samples were diluted (1/50) and tested in duplicate.

### 9. Clustering and statistical analyses

Continuous variables were expressed as the median (interquartile range) and compared using non-parametric tests (Mann-Whitney and Wilcoxon for non-paired and paired data, respectively), since the parameters were not normally distributed. Discrete variables were described as percentages (number of patients) and the chi-square or Fisher exact test was used as appropriate. Clustering of CFS and healthy individuals from immunophenotypic data was performed using the Cluster 3.0 software. Data were normalized according to medians and clustered using non-parametric correlations. Treeview 1.1 software was used to generate and visualize dendrograms.

### Example 1

### Quantification of main lymphocyte subsets

The absolute numbers of B cells (CD19⁺), NK cells (CD56⁺CD16⁺), T cells (CD3⁺), CD4 T cells (CD3⁺CD4⁺) and CD8 T cells (CD3⁺CD8⁺) in the CD45⁺ lymphocyte gate was similar between CFS individuals and healthy donors (data not shown). Similarly, the percentages of CD19⁺ cells, CD56⁺CD16⁺ and CD3⁺ cells in gated CD45⁺ lymphocytes showed similar values in both groups. *See* Fig. 1. However, significant differences were observed when the percentage of CD3⁺CD56⁺ lymphocytes was compared, being this population lower in the CFS group. This population encompasses a heterogeneous mix of effector T cells and NKT cells. A slightly unbalanced composition of the CD3⁺ cell subset was also observed, showing a higher CD4 T cell representation in the CFS group. *See* Fig. 1.

### Example 2

### NK cell phenotype and functionality assay

The phenotype of NK cells was evaluated by using a panel of antibodies against different activation markers and NK ligands. *See* Table 2; Brenu, 2011, *supra* and Lorusso L, et al., Autoimmun. Rev. 2009; 8:287-291. The three main NK cell subsets identified in the gating strategy (CD56^{high}CD16⁻, CD56⁺CD16⁺ and CD16⁺ CD56⁻cells) showed similar frequencies in control and CFS groups. Moreover, most of the markers analyzed showed comparable levels between groups; however, CD69, CD25 and NKp46 displayed significant different expression in the three subsets of NK cells. The expression of CD69 and NKp46 was higher in CFS individuals, while the expression of CD25, was surprisingly lower in this group. *See* Fig. 2.

A phenotypic feature of NK cells from CFS individuals and other debilitating infections is the low expression of CD57. *See* Stricker R, et al., Immunol. Lett. 2001; 76:43-48. The percentage of CD57 expressing NK cells is similar among groups, although the intensity of CD57 staining showed significantly lower values in CFS individuals. Despite phenotypic differences, NK cell activity assessed by the lysis of eGFP-K652cells using monocyte-depleted lymphocytes was similar in both groups at the different ratios assayed (p=ns, n=16). *See* Fig. 3. No major differences in NK cell spontaneous cell death could be detected between CFS individuals and healthy donors.

### Example 3

### T cell phenotype and functionality assay

T cells were stained with several antibody combinations previously described for the characterization of T cells in HIV infection. *See* Landay, 1991, and Massanella, 2010, *supra,* Appay V, et al., Cytometry A 2008; 73:975-983, and Deeks S, et al., Annu. Rev. Med. 2011; 62:141-155. CD4 T cells from CFS individuals and controls showed similar levels of naive (CD45RA⁺CCR7⁺CD28⁺), central memory (CD45RA⁻CCR7⁺CD28⁺) and effector memory (CD45RA⁻CCR7⁻CD28⁻) cells. However CD8 T cells showed a significantly lower frequency of effector cells (CD45RA⁻/⁺CCR7⁻CD28⁻CD27⁻). This event is probably referred with the lower level of CD56 expression observed in CD3⁺ cells. *See* Fig. 1. However, proliferative responses showed significant differences in CD4 but not in CD8 T cells between groups. No inter group differences in sensitivity to spontaneous cell death were noticed for CD4 and CD8 T cells. *See* Fig. 4.

To explore the reasons for the different behavior of CD4 and CD8 T cells, several markers of immunosenescence or immune exhaustion were analyzed. These markers showed discordant profiles: although CFS and control individuals had similar levels of the immunosenescence markers CD57 in CD4 and CD8 T cells, they showed markedly different expression patterns for the PD-1 and CD95 markers in CD4 and CD8 T cells, respectively. Finally, the frequency of Treg and the expression of activation markers were assessed also. Treg classic cells (CD4⁺CD25⁺FoxP3⁺) showed significantly higher percentages in CFS individuals, concomitant with lower levels of Ki67⁺ cells in non-regulatory CD4 T cells. In contrast, CD8 T cells only showed a trend in Ki67 positivity, although displayed higher expression of CD5, a marker associated with impaired T cell responses. *See* Hawiger D, et al., Immunity 2004; 20: 695-705 and Stamou P, et al., J. Immunol. 2003; 171:1278-1284. Lower levels of the activation marker CD38 in both the total CD8 T cell population and the memory CD45RO⁺ subset were observed in CFS individuals consistently. *See* Fig. 5. These data pointed out to an impaired T cell response in CFS individuals associated with increased Treg numbers and with some specific markers in both CD4 and CD8 T cells, not caused directly by a general status of immunosenescence.

### Example 4

### Sample clutersing

The data arising from the previous examples were collected and included in a database file. Parameters showing significant differences between CFS and control groups and some other parameters previously reported to distinguish both groups were selected for clustering analyses. Only 19 CFS and 25 controls healthy individuals were selected for the analysis, as various control individuals lacked some relevant data.

Parameters were analyzed individually for their ability to identify CFS individuals by calculating both their specificity and selectivity. This was done by ranking values and calculating false positive and false negative rates. False positive rate (FPR) was defined as the number of healthy individuals grouped in the CFS group, while false negative rate (FNR) was calculated as the number of CFS individuals clustered in the Control group. Specificity was calculated as (1- FPR)*100, while Selectivity was calculated as (1-FNR)*100. *See* Table 3.

**Table 3**

| | | | |
|---|---|---|---|
| Individual parameter values for clustering analysis | | | |

| Parameter | Specificity | Sensitivity | P value (CFS vs Ctrol) |
|---|---|---|---|
| CD69+ (% ofNK cells) | 74 | 80 | <0.0001 |
| NKp46+ (% ofNK cells) | 79 | 84 | <0.0001 |
| CD25+ (% ofNK cells) | 68 | 76 | 0.0012 |
| Ki67+(% of Cd4 T cells) | 63 | 72 | 0.0016 |
| CD56+ (% of T cells) | 68 | 68 | 0.0024 |
| Effector (% of CD8 T cells) | 63 | 72 | 0.0046 |
| CD57 (MFI in NK cells) | 58 | 68 | 0.0052 |
| Treg((% of Cd4 T cells) | 58 | 68 | 0.0155 |
| CD5-(% of Cd8 T cells) | 42 | 56 | 0.0188 |
| CD95+(% of CD8 T cells) | 58 | 68 | 0.0222 |
| CD38+ (% of CD45RO +CD8 T cells) | 68 | 76 | 0.0226 |
| CD4+(% of T cells) | 58 | 68 | 0.0297 |
| PD1+(% of Cd4 T cells) | 58 | 68 | 0.0380 |
| CD8 (% of T cells) | 58 | 68 | 0.0416 |
| CD57+ (% ofNK cells) | 53 | 64 | 0.1942 |

Selected parameters were included in a clustering analysis using the software Cluster version 3.0 (Michael Eisen, Stanford University, CA, US). The complete set of data was loaded and adjusted without log transformation. Values for each parameter were: i) centered to medians (i.e. median values were subtracted from the individual data, so that the median value of each parameter is 0), and ii) normalized (i.e. all values were multiplied by a scale factor S, so that the sum of the squares of the values in each row is 1.0 (a separate S is computed for each parameter)). Then, hierarchical clustering was done for parameters and individuals using Spearman-Rank correlations and a complete linkage as clustering method.

The clustering analysis generated in CDT format was visualized using the Tree View software version 1.1 (http://jtreeview.sourceforge.net, July 2012). Visualization settings were set in yellow/blue, where yellow squares correspond to positive values (above the median) and blue squares to negative samples (below the median), according to the following code:

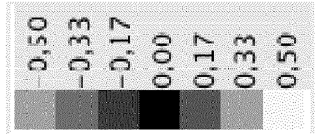

### Example 5

### T cell and NK cell phenotypes as markers for CFS

The association between the observed alterations in T and NK cell phenotypes in CFS individuals and its use as immunological markers for CFS was analyzed further. Poor correlations were observed between changes in the expression of CD25, CD69 or NKp46 in NK and increased Treg cells or CD38 and CD5 markers in CD8 T cells, suggesting that these immune cell subsets are modified in CFS individuals by different reasons. However, a cluster analysis yielded promising results as a CFS predictor. While unrestricted selection of markers provided poor selectivity, polishing marker selection according to their selectivity yielded a combination of 8 NK and T cell phenotypic markers that showed the best resolution in classifying CFS and healthy individuals (*p*=3.3x10⁻⁸). *See* Fig. 6. This combination showed a high sensitivity (100%) but a moderate false positive rate (5/24, specificity 79%). A more restrictive choice of parameters, including exclusively NK cell markers showed similar false positive rates (5/23, specificity 78%) but lower sensitivity (95%) in the detection of CFS cases. *See* Fig. 7.

### Example 6

### Comparative analysis

The method of the present invention and the assay described in Brenu, 2011, *supra,* were compared under several scenarios to analyze their relative specificity and sensitivity. Firstly, various parameters indicated in the Brenu paper as suggestive of CFS were evaluated according to the method of the present invention. In particular, the absolute counts of NK cell subtypes (i.e. CD56bright or CD56dimCD16+, and CD56-, Figure 4, Brenu, 2011, *supra*) and the relative frequency of NK cell subtypes (i.e. CD56bright or CD56dimCD16+ and CD56-, Brenu, 2011, *supra*) were assayed. Contrary to what was claimed in the Brenu paper, no significant differences were observed between CFS and healthy individuals.

Secondly, the specific predictive weight of the FoxP3+ marker, common to the Brenu paper and the method of the present invention, was evaluated. This was achieved by omitting the FoxP3+ parameter from the method of the invention (i.e. by using 7 instead of 8 biomarkers) to assess the individual predictive value of this specific marker. A modest loss of specificity (from a 79% to a 75%) and a concomitant loss of sensitivity (from 100% to 95%) were observed, suggesting that, although the FoxP3+ marker is helpful for identifying CFS patients, it is not a necessary parameter in the cluster. *See* Fig. 8.

In addition, the FoxP3+ marker was added to the first biomarker panel to assess any potential contribution it may have to increase the predictive capacity of the first panel. The FoxP3+ maker, by itself, did not improve the specificity and sensitivity of the first biomarker panel; rather a modest loss was observed in both parameters, from 82 to 74% and from 95% to 90%, respectively. This result suggested that the FoxP3+ marker alone did not increase the clustering of NK parameters and that; consequently, other T cell parameters, such as the elements of the second biomarker panel, are required for optimal clustering. *See* Fig. 9.

## Claims

1. A method for diagnosing CFS or a predisposition thereto *in vitro* which comprises:
a) determining the levels of the CD25, CD69, NKp46 and CD57 biomarkers in CD56+CD16+ NK cells in a sample obtained from a subject, and
b) comparing the levels obtained in a) with a reference value for each marker, wherein increased levels of CD69 and NKp46 and decreased levels of CD25 and CD57 with respect to the reference value for each marker is indicative that the subject suffers or is predisposed to suffering CFS.

2. The method method according to claim 1 further comprising:
a) determining the value of at least one parameter selected from the group consisting of :
i) the level of CD56 in CD3+ T cells,
ii) the frequency of Tregs in CD4+ T cells,
iii) the level of Ki67 in CD4+ T cells,
iv) the level of CD5 in CD8+ T cells, and
v) the frequency of effector cells in CD8+ T cells, and
b) comparing the values obtained in step a) with a reference value for each of the parameters,
wherein increased levels of CD5, increased frequency of Tregs in CD4+ cells, decreased levels in CD56, decreased levels of Ki67 or decreased frequency of effector cells with respect to the reference values marker is indicative that the subject suffers or is predisposed to CFS.

3. The method according to claim 2 wherein the levels of CD25, CD69, NKp46 and CD57 in CD56+CD16+ NK cells, the levels of CD56 in CD3+ T cells, the frequency of Tregs in CD4+ cells, the levels of Ki67 in CD4+ cells and the frequency of effector cells in CD8+ T cells are determined.

4. The method according to claim 2 wherein the levels of CD25, CD69, NKp46 and CD57 in CD56+CD16+ NK cells, the levels of CD56 in CD3+ T cells, the levels of Ki67 in CD4+ cells and the frequency of effector cells in CD8+ T cells are determined.

5. The method according to any of claims 1 to 4 wherein the sample is a body fluid.

6. The method according to claim 5 wherein the body fluid is blood.

7. The method according to any of claims 1 to 6 wherein the levels are determined by flow cytometry.

8. A kit comprising reagents for assaying the levels of CD25, CD69, NKp46 and CD57 in CD56+CD16+NK cells.

9. The kit according to claim 8 further comprising reagents for assaying the levels of at least one marker selected from the group consisting of the level of CD56 in CD3+ T cells, the frequency of Tregs in CD4+ T cells, the level of Ki67 in CD4+ T cells, the level of CD5 in CD8+ T cells and the frequency of effector cells in CD8+ T cells.

10. The kit according to claim 9 comprising reagents for determining the levels of CD25, CD69, NKp46 and CD57 in CD56+CD16+ NK cells, the levels of CD56 in CD3+ T cells, the frequency of Tregs in CD4+ cells, the levels of Ki67 in CD4+ cells and the frequency of effector cells in CD8+ T cells.

11. The kit according to claim 9 comprising reagents for determining levels of CD25, CD69, NKp46 and CD57 in CD56+CD16+ NK cells, the levels of CD56 in CD3+ T cells, the levels of Ki67 in CD4+ cells and the frequency of effector cells in CD8+ T cells.

12. The kit according to any of claims 8 to 11 wherein the reagents are antibodies.

13. Use of a kit according to any of claim 8 to 12 for the diagnosis of CFS or of a predisposition thereto.

14. A reagent for inhibiting the expression or activity of at least one biomarker selected from the group consisting of CD69, NKp46 and CD5 for use in the treatment or prevention of CFS in a subject in need thereof.

15. The reagent according to claim 14 further comprising a compound for inhibiting the frequency of Tregs in CD4+ cells.

16. A reagent for inducing the expression or activity of at least one biomarker selected from the group consisting of CD25, CD57, CD56 and Ki67 for use in the treatment or prevention of CFS in a subject in need thereof.

17. A composition or kit-of-parts comprising the reagent according to any of claims 14 to 16.
